(19) Europäisches Patentamt
European Patent Office
Office européen des brevets

(11) EP 4 667 939 A1

(12) EUROPEAN PATENT APPLICATION

(43) Date of publication:
**24.12.2025 Bulletin 2025/52**

(21) Application number: **24305986.2**

(22) Date of filing: **21.06.2024**

(51) International Patent Classification (IPC):
***G01N 33/68** (2006.01)* ***C07D 231/54** (2006.01)*
***C07D 271/04** (2006.01)* ***C07D 413/12** (2006.01)*
***C07D 495/04** (2006.01)* ***C12Q 1/6886** (2018.01)*

(52) Cooperative Patent Classification (CPC):
(C-Sets available)
**C12Q 1/6886; G01N 33/6848;** C07D 271/04;
C07D 413/12 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Commissariat à l'Energie Atomique et aux Energies Alternatives**
**75015 Paris (FR)**

(72) Inventors:
- **TARAN, Frédéric**
  **91191 Gif sur Yvette Cedex (FR)**
- **TRUILLET, Charles**
  **91400 Orsay (FR)**
- **PRUVOST, Alain**
  **91191 Gif sur Yvette Cedex (FR)**
- **RIBERAUD, Maxime**
  **91400 Saclay (FR)**

(74) Representative: **Santarelli**
**Tour Trinity**
**1 bis Place de la Défense**
**92400 Courbevoie (FR)**

(54) **MULTIPLEX CANCER CELL PROFILING USING MS-TAGGED PROBES CONTAINING AN IMINOSYDNONE CLEAVABLE LINKER**

(57) The accurate quantification of biomarkers is paramount in modern medicine, particularly in cancer where precise diagnosis is imperative for targeted therapy selection. There is therefore a long felt need for techniques using cell surface specific agents that can be used for identifying cancerous tissue and therefore the need for cell surface specific biomarkers. In this context, the present invention proposes performant multiplexed analysis methods using probes linked to cleavable MS-tag isotopologues by a click-and-release bioorthogonal reaction involving an iminosydnone linker. The great potential of this approach is herein demonstrated in culture cells, in tissues as well as *in vivo*, thereby unveiling promising diagnostic avenues, especially for cancer cell immunoprofiling.

EP 4 667 939 A1

(52) Cooperative Patent Classification (CPC): (Cont.)

C-Sets
**C12Q 1/6886, C12Q 2523/107, C12Q 2525/205, C12Q 2537/143, C12Q 2563/167, C12Q 2565/102**

## Description

### SUMMARY OF THE INVENTION

The accurate quantification of biomarkers is paramount in modern medicine, particularly in cancer where precise diagnosis is imperative for targeted therapy selection. There is therefore a long felt need for techniques using cell surface specific agents that can be used for identifying cancerous tissue and therefore the need for cell surface specific biomarkers. In this context, the present invention proposes performant multiplexed analysis methods using probes linked to cleavable MS-tag isotopologues by a click-and-release bioorthogonal reaction involving an iminosydnone linker. The great potential of this approach is herein demonstrated in culture cells, in tissues as well as *in vivo,* thereby unveiling promising diagnostic avenues, especially for cancer cell immunoprofiling.

### DECRIPTION OF THE PRIOR ART

Extensive efforts have been made in the recent years to reduce side effects of cancer therapies by developing targeted treatment strategies based on cell receptors overexpressed within tumors. Among the possible targeted therapies, those employing monoclonal antibodies (mAbs) are certainly the most important to date [1]. Several therapeutic mAbs are routinely used for cancer immunotherapies, a dozen of antibody-drug conjugates have been already approved by the FDA and many others are advancing through the pipeline, promising enhanced treatment options across various cancer types [2]. It is thus very likely that in the coming years a considerable number of targeted therapies will be available, which is excellent news for patient health care but raises the problem of selecting the best therapy among those available. Furthermore, the levels of overexpressed protein receptors not only differ within the cancer types ($10^4$ to $10^6$ receptors per cell) but also vary within the different patient tumor lesions and also from patient to patient [3]. In this context, the molecular analyses of cancer cells involving quantification of a maximum number of protein receptors is crucial in establishing precise diagnoses for choosing and guiding available treatments [4].

Among the described methods used to quantify the abundance of protein receptors in cells and in biopsy tissues [5], cyclic immunofluorescence appears as one of the most promising. This approach involves the use of fluorescent antibodies which, after incubation and staining, need to be de-stained to allow a new cycle of detection with new fluorescent antibodies. Several approaches have been explored to inactivate the fluorophores attached to antibodies. Photobleaching [6] and the use of harsh chemical agents (NaOH-$H_2O_2$ for example) [7] are effective approaches but these conditions may induce degradation of biological material and often result in interference with subsequent cycles of staining. DNA displacement has also been successfully investigated to erase fluorescence but nonspecific binding between DNA and endogenous biomolecules may lead to increased background [8].

The use of click chemistry has recently emerged as a powerful additional strategy for mild and robust cyclic immunofluorescence. The group of Guo has notably successfully used fluorescent antibodies constructed with an azide-based linker that can be cleaved upon addition of a phosphine to remove the fluorescent label [9]. More recently, Carlson, Weissleider et al. exploited the powerful inverse electron demand Diels-Alder reaction to quench fluorescent antibodies derivatized by trans-cyclooctenes with tetrazine quenchers [10]. Yet, these techniques cannot be used *in vivo* to detect or quantify cells of interest in their natural environment, in human or animal bodies. As a matter of fact, they can only be used *ex vivo* to detect biomarkers by using cyclic immunofluorescent analysis of stained tissue slices or cells obtained from biopsies. As only immunofluorescence devices are involved, the results are not quantitative and no more than 4 biomarkers can be detected per cycle.

There is therefore still a need for new techniques using cell surface specific agents that can be used *in vivo* for identifying more specifically and reliably a high number of biomarkers, characteristic of cells, tissues of interest, or particular diseases, especially in situ in human bodies.

### DETAILLED DESCRIPTION OF THE INVENTION

The present inventors herein report a new strategy for multiplexed protein analysis of cells (preferably cancer cells) and tissue biopsies using cleavable antibodies labelled with mass spectrometry enhancers (also herein called "MS-tags") (see Figure 1B).

More precisely, the MS-tags have been covalently coupled to antibodies of interest by means of the click-and-release bioorthogonal reaction described in WO2015/193455, the content of which is herein wholly incorporated by reference. Accordingly, the antibodies of the invention are coupled to the MS-tag through an iminosydnone cleavable compound which can be cleaved as disclosed in WO2015/193455 with an alkynyl cleaving compound. This alkynyl cleaving compound can be administered *in vivo,* releasing the cleaved MS-tags in naturally excreted fluids where they can be easily identified and quantified. Moreover, the alkynyl compound remains attached to the iminosydnone compound and thus to the bound antibodies, allowing the localisation of the antigens recognized by the antibodies, where and if they are

expressed by the target cells.

In a proof of concept study, the present inventors demonstrated the feasibility of this approach by quantifying four different receptors in a single analysis on culture cells, on *ex vivo* tissues as well as *in vivo* (administration of the antibodies in mice bearing tumors). The results described below prove that it is possible to characterize tumors in their native environment and without the need for tissue removal.

The main advantages of the present invention are the possibility to use complex probes cocktails allowing multiplexed analysis, and to quantify the *in situ* level of antigenic expression thanks to detectable and quantifiable MS-tags that can be used *in vivo* and naturally excreted in physiological fluids (e.g., urine). In addition, as the chemistry behind this technology allows bioorthogonal mild detachment of the tags from the bound probes, an infinite number of cycles is theoretically possible. For these reasons, the present invention is a valuable new tool to identify and quantify the expression of a tremendous number of different cells antigens or receptors, for example to help tumor characterization and the selection of appropriate immunotherapies. Importantly, the methods and uses of the invention can be performed *in vivo* and are not limited by the known limitations of the immunofluorescence techniques.

MS-tagged probes of the invention

In a first aspect, the present invention targets a probe specifically recognizing a target biological molecule, said probe being linked to a detectable mass spectrometry enhancer (MS-tag) through a cleavable linker (CL). This "MS-tagged probe" is hereafter referred to as the "probe of the invention". This probe therefore contains i) a probe part (PP) specifically recognizing a target biological molecule, ii) a cleavable linker (CL) and iii) a MS-tag.

The different parts of the probe of the invention will now be described in more details.

*Cleavable linker CL*

In the probe of the invention, the cleavable linker CL is preferably an iminosydnone compound (also called "sydnonimine") having the formula I as defined in WO2015/193455:

(I) 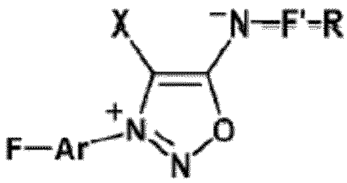

Wherein :

X is selected from the group consisting of a hydrogen atom, a halogen atom, an aryl diazo group, an alkyl group, an aryl group, an alkenyl group, an alkynyl group, an alkoxy group, a thioether group and an amino group,

F is a functional group selected from the group consisting of: a carboxylic acid COOH group, a thiol SH group, a maleimide group, an activated ester, a halogen atom, an alkene or alkyne group (optionally interrupted by at least one heteroatom selected among O, N and S), an amino (-NRR') group wherein R and R' are independently hydrogen atoms, alkyl, alkene, alkyne or aryl groups, a hydroxylamine ($-ONH_2$) group, a hydrazine ($-NH-NH_2$) group, an azido ($-N_3$) group, a diazonium ($-N_2^+$) group, optionally in presence of a counterion, a boronic acid - $B(OR'')_2$ group wherein R" is a hydrogen atom or an alkyl group, an isocyanate (-N=C=0) or isothiocyanate (-N=C=S) group, a chlorosulfonyl ($-SO_2Cl$) group, a -C=C-C=N group, an aldehyde CHO group, a ketone COR‴ group wherein R‴ is an alkyl group, and an alkyl group substituted by at least one of said groups,

F' is a carbonyl group (C=0), a sulfonyl group ($SO_2$), an alkyl, an aromatic ring (as proposed in [11]) or a phosphoryl group (P=0),

R is selected from an optionally substituted aryl group, an optionally substituted alkyl, alkenyl or alkynyl group, an optionally substituted alkoxy or aralkyloxy group, an optionally substituted thioether group, an optionally substituted amino group, wherein the alkyl, alkenyl and/or alkynyl groups may be interrupted by at least one heteroatom selected from nitrogen, oxygen and sulphur atoms and wherein said substituents are one or more groups selected from a carboxylic acid COOH group, a thiol SH group, a maleimide group, an activated ester, a halogen atom, an alkene or alkyne group, optionally interrupted by at least one heteroatom selected among O, N and S, an amino (-NRR') group, wherein R and R' are independently hydrogen atoms, alkyl, alkene, alkyne or aryl groups, a hydroxylamine ($-ONH_2$)

group, a hydrazine ($-NH-NH_2$) group, an azido ($-N_3$) group, a diazonium ($-N_2^+$) group, optionally in presence of a counterion, a boronic acid $-B(OR")_2$ group, wherein R" is a hydrogen atom or an alkyl group, an isocyanate (-N=C=0) or isothiocyanate (-N=C=S) group, a chlorosulfonyl ($-SO_2Cl$) group, a -C=C-C=N group, an aldehyde CHO group, a ketone COR‴ group, wherein R‴ is an alkyl group, or a linker bearing at least one of the above substituents, said optional linker comprising preferably any hydrocarbon chain comprising C and H atoms and optionally O and/or N and/or S atoms, such as PEG molecules, a $-(CH_2)_q(CH_2O)_r-COO-(CH_2)_{q'}(CH_2O)_{r'}$ group, a $-(CH_2)_q(CH_2O)_r-NH-C(=0)-(CH_2)_{q'}(CH_2O)_{r'}$ group, a $-(CH_2)_q(CH_2O)_r-CONH-(CH_2)_{q'}(CH_2O)_{r'}$ group, wherein q, q', r and r' are integers independently ranging from 0 to 10, or a triazolyl group, for instance,
and

Ar is an alkyl or NR2 group with R is an alkyl, an aryl, an acyl or a sulfonyl group, or an optionally substituted aromatic group, preferably selected from the group consisting of: a phenyl group; a heteroaromatic group, such as pyridine, thiophene, imidazole, thiazole, pyrazole, pyrole or furane; and a polyaromatic group, such as anthracene or phenanthrene. The phenyl, heteroaromatic and/or polyaromatic group may be substituted with at least one substituent (in addition to the F group when the aromatic is substituted with a F group), preferably selected from the group consisting of a halogen atom, an alkyl group, an alkoxy group, a carboxyl COOH group, a $COOR_3$ group, wherein $R_3$ is an alkyl group, and a nitro $NO_2$ group. In an embodiment, the optionally substituted aromatic group is an optionally substituted phenyl group, preferably a non-substituted phenyl group.

In the above definitions of F and R, the counterion can be any ion appropriate for compensating the charge of the diazonium group, and may be easily chosen by anyone of ordinary skill in the art. For instance, the counterion may be selected from the group consisting of halogenates, $BF_4$, $NO_3$; $HSO_4^-$, $PF_6^-$ CH3COO-, $N(SO_2CF3)_2^-$, $CF_3SO_3^-$, $CH_3SO_3^-$, $CF_3COO^-$ , $(CH_3O)(H)PO_2^-$ and $N(CN)_2^-$.

The compounds F and R are covalently coupled on a one hand to the probe and on the other hand, to the mass tag, respectively. In a preferred embodiment, PEG spacers are introduced into the iminosydnone core (e.g., between Ar and F and/or between R and the MS-tag) as described in the compounds disclosed in Figures 2, 3 and 5.

In an embodiment, X is a halogen atom or a hydrogen atom.

When Ar is an optionally substituted phenyl group, the functional group F is in *meta* or *para* position of the phenyl group, preferably in *para* position. When Ar is an optionally substituted heteroaromatic or polyaromatic group, the functional group F can be in any position of the aromatic group.

In a preferred embodiment, the functional group F is selected from the group consisting of: a carboxylic acid COOH group, a thiol SH group, a maleimide group, an activated ester, a disulfide rebridging reagent such as pyridazine diones, an amino (-NRR') group, wherein R and R' are independently hydrogen atoms, alkyl, alkene, alkyne or aryl groups as defined above, a hydroxylamine ($-ONH_2$) group, a hydrazine ($-NH-NH_2$) group, an azido ($-N_3$) group, an isocyanate (-N=C=0) or isothiocyanate (-N=C=S) group, a chlorosulfonyl ($-SO_2Cl$) group, a -C=C-C=N group, and an alkyl group substituted by at least one of the groups above. In a highly preferred embodiment, the functional group F is selected from the group consisting of a carboxylic acid COOH group, an activated ester, such as a N-hydroxysuccinimide, a N-hydrophthalimide ester, a perfluorinated ester or an acylurea, and an alkyl group substituted by at least one of these groups. In a more preferred embodiment, the functional group F can bind covalently to the probe by conventional means.

In another preferred embodiment, F' is a C=0 group and R is an alkoxy group such as a tert- butoxy group. Preferably, the R alkoxy group is bonded through the oxygen atom to the C=0 F' group, thus forming a carbamate moiety.

In a more preferred embodiment, F' is a C=0 group and R is an amino group. Preferably, the R amino group is bonded through the nitrogen atom to the C=0 F' group, thus forming a urea moiety. In a preferred embodiment, F' is a C=0 group, R is an amino group forming a urea, and X is a hydrogen atom as disclosed on Figures 2 and 3 and 5.

In a preferred embodiment, Ar is a phenyl group and F is an activated carboxylic acid.

Examples of iminosydnones of formula (I) that may be used as a linker in the probe of the invention are selected from the group consisting of:

- (2-(4-hydroxyphenyl)- 1 ,2,3-oxadiazol-2-ium-5-yl)(p-tolylcarbamoyl)amide ;
- (2-(4-carboxyphenyl)-1,2,3-oxadiazol-2-ium-5-yl)(p-tolylcarbamoyl)amide ; and
- ((4-(ethoxycarbonyl)phenyl)carbamoyl)(2-(4-hydroxyphenyl)-1,2,3-oxadiazol-2-ium- 5-yl)amide ;
- (3-(4-carboxyphenyl)-1,2,3-oxadiazol-3-ium-5-yl)(methylcarbamoyl)amide.

The racemic forms, tautomers, enantiomers, diastereoisomers, epimers, solvates and salts of the compounds used in the processes according to the invention are also part of the scope of the invention.

As examples of salts may be cited acid addition salts, such as hydrochloric, hydrobromic, hydroiodic, phosphoric, and the like. Representative examples of suitable organic acids include formic, acetic, trichloroacetic, trifluoroacetic, propionic, benzoic, cinnamic, citric, fumaric, maleic, methanesulfonic and the like. Further examples of pharmaceutically

acceptable inorganic or organic acid addition salts include the pharmaceutically acceptable salts listed in J. Pharm. Sci. 1977, 66, 2, and in Handbook of Pharmaceutical Salts: Properties, Selection, and Use edited by P. Heinrich Stahl and Camille G. Wermuth 2002.

Methods to produce such compounds and all the compounds encompassed herein are well described in WO2015/193455 (incorporated herein by reference) and/or in the examples below.

*MS-tags*

The MS-tag bound to the probe of the invention can be any MS-tag that can be used in mass spectrometry analysis, e.g., in LC-MS, LC-MS/MS, MALDI-MS, MALDI-MS/MS, MALDI-TOF-MS, TOF-TOF-MS, etc. Exemplary mass tags that can be used herein are disclosed in WO95/04160, WO98/26095, WO97/27327, WO97/27325, WO97/27331, WO01/68664 and WO03/025576. These applications disclose tags that comprise polyamide compounds, essentially peptides or peptide-like tags, which means that these tags can be prepared using a number of peptide synthesis methods that are well known in the art. In addition, the use of peptide and peptide-like tags enables coupling of these tags to oligonucleotides using a variety of peptide conjugation techniques that are known in the art. Other mass tags particularly suitable for the present invention are an isobaric mass tag. Such mass tags are disclosed for example in WO01/68664 and WO03/025576.

Other possible tags are guanidinium salts ([12]), $\alpha$-cyano-4-hydroxycinnamic acid (HCCA) ([13]) or acylcarnitine ([14]).

The choice of the MS-tag is important as this tag may impact the signal detected by MS analysis ($10^2$ - to $10^3$ - fold depending on the type of tag), and the possibilities of isotope incorporation, thus on multiplexed analyses capacities.

In a preferred embodiment, the MS-tag used in the present invention is therefore tris(2,4,6,-trimethoxyphenyl)-phosphonium (TMPP) or an isotope thereof, as disclosed in the examples below. Because of its moderate hydrophobicity and the presence of a permanent positive charge, TMPP is known to be highly suitable for proteomic studies using MALDI analysis. Importantly, TMPP has 9 methoxy groups, 6 aromatic hydrogens and 27 carbon atoms in its structure which represent as many possibilities for deuterium and carbon 13 labelling. In total there are 60 atoms that can be isotopically labelled, which gives the possibility to theoretically generate 61 TMPP-isotopologues. Thus, the multiplex potential of using TMPP as MS-tag in the probes of the invention is very high, as it allows the multiplexed detection of 70 biomarkers in one single LC-MS/MS analysis. Moreover, it can be safely administered in animals and is excreted naturally in physiological fluids such as urine (see the examples below).

The synthesis of these preferred compounds has been described in the art and is reminded in the examples below (see also figure 4). In the context of the invention, the inventors have produced four isotopologues of TMPP, namely TMPP-H33, H24, H15 and H6, and have linked them to the cleavable linker described above (see examples below and figure 3). Four antibodies according to the invention - each linked to one of this TMPP isotopologue - have been successfully generated and used *in vitro* and *in vivo,* so as to demonstrate the feasibility and the efficiency of the proposed invention.

The MS-tags (preferably TMPPs) can be attached to the cleavage linker using standard peptide coupling.

*Probe part*

The probe part of the probe of the invention typically contains a biological molecule (e.g., a protein, nucleic acid, aptamer, etc.) that specifically interacts with or specifically binds to, and thus detects, a target biological molecule such as a protein, an oligonucleotide, a non-peptidic chemical molecule, a small organic molecule, or another cellular component. Nonlimiting examples of biological molecules that specifically interact with or specifically bind to such targets and can therefore constitute the probe part of the probe of the invention include: nucleic acids (e.g., oligonucleotides), proteins (e.g., antibodies, transcription factors, zinc finger proteins, non-antibody protein scaffolds, etc.), and aptamers.

In a preferred embodiment, the probe part of the probe of the invention is an antibody.

By **"antibody",** it is herein meant a molecule comprising at least one binding domain for a given antigen and a constant domain comprising an Fc fragment capable of binding to Fc receptors (FcR). In most mammals, like humans and mice, an antibody consists of four polypeptide chains: two heavy chains and two light chains bound together by a variable number of disulfide bridges providing flexibility to the molecule. Each light chain consists of a constant domain (CL) and a variable domain (VL); the heavy chains consisting of a variable domain (VH) and three or four constant domains (CH1 to CH3 or CH1 to CH4) according to the isotype of the antibody. In a few rare mammals, such as camels and llamas, the antibodies consist of only two heavy chains, each heavy chain comprising a variable domain (VH) and a constant region. The variable domains are involved in antigen recognition, while the constant domains are involved in the biological, pharmacokinetic and effector properties of the antibody.

Antibodies include, but are not limited to, synthetic antibodies, monoclonal antibodies, recombinantly produced antibodies, multispecific antibodies (including bi-specific antibodies), human antibodies, humanised antibodies, camelised antibodies, chimeric antibodies, intrabodies, anti-idiotypic (anti-Id) antibodies, and functional fragments of any of the above, which refers a portion of an antibody heavy or light chain polypeptide that retains some or all of the biological function of the antibody from which the fragment was derived. The antibodies provided herein can be of any type (e.g., IgG, IgE, IgM, IgD, IgA and IgY), any class (e.g., IgG1, IgG2, IgG3, IgG4, !gA1 and IgA2), or any subclass (e.g., IgG2a and IgG2b) of immunoglobulin molecule. The **"functional fragments"** can be selected, without limitation, in the group consisting of Fab, Fab', $(Fab')_2$, Fv, scFv (sc for single chain), Bis-scFv, scFv-Fc fragments, $Fab_2$, $Fab_3$, minibodies, diabodies, triabodies, tetrabodies, and nanobodies, and fusion proteins with disordered peptides such as XTEN (extended recombinant polypeptide) or PAS motifs, and any fragment of which the half-life time would be increased by chemical modification, such as the addition of poly(alkylene) glycol such as poly(ethylene) glycol ("PEGylation") (pegylated fragments called Fv-PEG, scFv-PEG, Fab-PEG, F(ab')2-PEG or Fab'-PEG) ("PEG" for Poly(Ethylene) Glycol), or by incorporation in a liposome, said fragments having at least one of the characteristic CDRs of the antibody according to the invention.

The broad range of reactive chemical groups on antibodies makes them amenable to various conjugation methods. In addition to conventional methods for modifying amino acids via free amines and carboxyl groups, modifications at selectively reduced cysteine residues and glycosylation sites are commonly used for functionalising antibodies. Unlike modifications to lysine and acidic amino residues, these site-directed modifications are less likely to lead to loss of activity from changes to the antigen-binding (Fab) region. Alternatively, antibodies can be engineered to incorporate non-natural amino acids to allow site-specific conjugations ([15]).

The antibody of the invention can be any antibody that recognizes specifically a cellular biomarker. This biomarker can be present at the surface of the cells (extracellular surface biomarker), or inside the cells (intracellular). In a particular embodiment, this biomarker is involved in the characterization of at least one disease. This disease can be cancer, autoimmune disorder, or any other disease. Accordingly, the MS-tagged antibodies of the invention can be used to diagnose or monitor the progression of any of these diseases.

The probes of the invention preferably recognize target any biological molecule of interest whose presence or absence is specific to a disease (hereafter also called "biomarkers"). In the context of the invention, target biological molecules are in particular biomarker proteins, oligonucleotides (such as DNAs or RNAs), non-peptidic small chemical molecules and/or cellular components whose presence or absence is specific to the onset or the stage of a disease. The MS-tagged probes of the invention are therefore useful to diagnose or monitor the progression of the corresponding disease (see below).

As disclosed in the examples below, the probes of the invention are preferably antibodies recognizing target biomarker proteins. These biomarker proteins are more preferably located at the surface of tumor cells. These MS-tagged antibodies are therefore useful to diagnose or monitor the progression of tumours.

The significant presence of VEGF-A, HER2, EGFR, and PDL1 proteins at the surface of tumoral cells has been evidenced by numerous studies. In a more preferred embodiment, the antibodies of the invention can therefore recognize specifically any of these biomarkers. Accordingly, the antibodies of the invention would be for example: Trastuzumab (TRZ, anti-Her2), Cetuximab (CTX, anti-EGFR), Durvalumab (DUR, anti-PDL1) and Bevacizumab (BVZ, anti-VEGF). Other antibodies that have been developed and granted in cancer therapies are listed for example in Table 1 of [16], and could be use as probe part in the probes of the invention.

*Cleaving compound*

As demonstrated in WO2015/193455, the cleavable linker CL described above can be easily cleaved upon contact with a strained alkyne. This strained alkyne will be herein referred to as the "cleaving compound" of the invention. The inventors herein show that the use of this cleaving compound is very advantageous, as it enables to cleave the cleavable linker *in vitro* as well as *in vivo* when injected *in vivo,* and it remains attached to the probe and can therefore convey a label (e.g., a fluorochrome) in order to label the probe when bound to its target biomarker (figure 8).

By "strained alkyne", it is herein meant a cyclic alkyne, in particular cyclooctynes such as bicyclo- [6.1.0]-nonyne (BCN), 3,3-difluorocyclooct-1-yne (DIFO), dibenzocyclooctyne (DBO) or cycloheptynes such as tetramethylthiacycloheptyne (TMTH). It is preferably dibenzoazacyclooctyne (DBCO), as disclosed in the examples below. These compounds are herein designated as "the cleaving compound of the invention". They are biocompatible and can therefore be injected in animals (including in humans).

The iminosydnones disclosed above react very efficiently with these strained alkynes. As explained in WO2015/193455, the coupling reaction implies the formation of a cycloadduct formed by a [3+2] cyclization turning to a stable pyrazole by retro-Diels Alder reaction. In the context of the invention, a cycloadduct including the probe part of the probe of the invention is generated, and the released product contains the MS-tag (figure 5B).

In a preferred embodiment, the cleaving compound of the invention is covalently coupled or associated to a detectable label. In this case, the detectable label will remain bound to the cycloadduct and thus to the probe part of the probe of the invention, after the reaction between the iminosydnone and the cleaving compound occurs.

This **detectable label** is herein advantageously used to track the localization of the biomarker proteins *in situ* or in biological samples collected from the patient (e.g., on tumor biopsies). Commonly used detectable labels are for example:

- Fluorescent Dyes/Probes such as Fluorescein (FITC), Rhodamine, Alexa Fluor dyes, Cyanine dyes (e.g., Cy3, Cy5), GFP (Green Fluorescent Protein) and other fluorescent proteins (e.g., RFP, YFP), DAMRA, etc.
- Radioisotopes: Tritium ($^3H$), Carbon-14 ($^{14}C$), Phosphorus-32 (32P), Sulfur-35 ($^{35}S$) Iodine-125 ($^{125}I$), fluor-18 ($^{18}F$), carbon-11 ($^{11}C$), zirconium-89 ($^{89}Zr$), Copper-64 ($^{64}{}_{Cu}$)
- Enzymatic Tags: Horseradish peroxidase (HRP), Alkaline phosphatase (AP), β-Galactosidase (β-gal), Acetylcholinesterase (AChE), Luciferase (for bioluminescence)
- Biotin: A small molecule that has a very strong affinity for streptavidin or avidin, which can be conjugated to an enzyme or fluorescent dye to produce a signal.
- Colloidal Gold: Particles used especially in electron microscopy and immunochromatographic assays (e.g., lateral flow assays).
- Quantum Dots: Semiconductor nanocrystals with unique optical properties that make them useful as fluorescent probes in imaging and assays.
- Chemiluminescent Labels: Luminol, Aequorin
- Heavy Atoms for X-ray Crystallography: Selenomethionine
- Mass Tags for Mass Spectrometry: Isobaric Tags for Relative and Absolute Quantitation (iTRAQ), Tandem Mass Tags (TMT)
- Magnetic Beads: Micro- or nano-sized particles that can be used for separation and detection of labeled targets using magnetic fields.
- Chromogenic Substrates: Tetramethylbenzidine (TMB) for HRP; p-Nitrophenyl phosphate (pNPP) for AP
- Haptens: DNP (dinitrophenol), DIG (digoxigenin)
- Spin Labels: Nitroxide radicals or other paramagnetic molecules used in electron paramagnetic resonance (EPR) spectroscopy to study the dynamics of the labeled molecules.
- Photostable Dyes: Dyes that resist photobleaching and retain their fluorescence over time, useful for live-cell imaging and long-term studies
- Energy Transfer Pairs: Molecules that participate in Förster resonance energy transfer (FRET) or bioluminescence resonance energy transfer (BRET), enabling the study of molecular interactions.

If the cleaving compound is intended to be administered to animals (in particular to human beings), radioactive detectable labels such as Fluor-18 shall be preferred.

The choice of a label depends on the specific requirements of the experiment, including sensitivity, specificity, ease of detection, and the potential for interfering with the function of the molecule being studied. In the context of the invention, the detectable label is preferably a fluorophore, so that it will be possible to detect the bound probe easily with fluorescent microscopic devices or flow cytometry.

Kits of the invention

The present invention also relates to kits containing the probes of the invention, as described above, and instructions for using them.

Preferably, the kits of the invention are used in multiplex assays and therefore contain several sets of probes that are specific to different target biomarker molecules (each set of probes enabling to identify one target biomarker molecule). Accordingly, all the probes linked to the same detectable MS-tag bind to the same biomarker molecule (representing a "set of probes" according to the invention), so that the detection of said particular MS-tag in a sample by the appropriate mass spectrometry device will allow to identify and/or quantify the presence of the said biomarker molecule in the sample.

For example, one kit according to the invention may contain:

- a set of antibodies recognizing the biomarker protein A, said antibodies being coupled through the cleavable linker of the invention to a MS-tag having a particular mass-to-charge ratio ($m_A/z_A$) which will be the signature of the presence of A,
- a set of antibodies recognizing the biomarker protein B, said antibodies being coupled through the cleavable linker of the invention to a MS-tag having a particular mass-to-charge ratio ($m_B/z_B$) which will be the signature of the presence of B,
- a set of antibodies recognizing the biomarker protein C and coupled through the cleavable linker of the invention to a MS-tag having a particular mass-to-charge ratio (mc/zc) which will be the signature of the presence of C,
- etc.

In other words, the probes from different sets of the kit of the invention differ i) by their biomarker targeting specificity and ii) by the TMPP isotopologues that are bound thereto.

In a second aspect, the present invention therefore targets a kit containing at least two sets of probes as defined above, wherein the probes within each set of antibodies specifically recognize the same target biomarker molecule, and are linked to the same MS-tag whose mass-to-charge ratio is specifically associated to said target biomarker molecule.

In a preferred embodiment, this kit may contain between 2 and 100 different sets of such probes, more preferably between 5 and 90 different sets of such probes, even more preferably more than 10 different sets of such probes.

In a preferred embodiment, the MS-tags linked to the probes in the kit of the invention are TMPP isotopologues as disclosed above. In the kit of the invention, all the probes within each set target the same biomarker and are linked to the same TMPP isotopologue. Probes from different sets therefore differ by their biomarker targeting specificity and the TMPP isotopologues that are bound thereto.

In the kit of the invention, the different sets of probes can be all contained within the same container, or they can be displayed separately in different containers.

In a preferred embodiment, the kit of the invention further contains, in a different container, an alkynyl cleaving compound as described above. In a more preferred embodiment, this cleaving compound is DBCO. In an even more preferred embodiment, the alkynyl cleaving compound is labelled with a detectable label, for example with a fluorochrome.

The kit of the invention furthermore preferably contains instructions for using these compounds according to the use / methods of the invention (as described below) and the biomarkers at stake.

Diagnostic uses and methods of the invention

The inventors herein show that antibodies according to the invention interact efficiently with their target biomarker in biological media, such as culture media, cell lysates or tissue sections. Moreover, the cleavable linker is efficiently cleaved by the cleaving compound of the invention, and the amount of the released tags measured in a biological sample of the patient precisely correlates to the amount of biomarkers theoretically expressed *in situ* by the target cells (figures 6-8). These results show that the system of the present invention, including the particular probes and the biocompatible cleaving compound as described above, can be used *in vivo* or *in vitro* to detect several biomarkers in one single process.

Therefore, the probes and the kit of the invention, as defined above, are useful to detect biomarker molecules expressed by cells of interest. These biomarkers molecules can be any molecules (proteins, DNAs, RNAs, small chemical molecules, etc.) whose expression or lack of expression is known to be specifically associated to a defined disease or disorder. This molecule can be either surficial (i.e., expressed at the cell surface so as to be reached by the probes of the invention without having to permeabilize the cells), or intracellular (in this case, permeabilizing the cell surface or performing a cell lysis will be required). The biomarker of the invention can even be a circulating molecule, i.e., a protein, DNA or RNA which is not bound to a cell (acellular biomarker).

*In vitro*

The probes and the cleaving compounds of the invention are preferably added *in vitro* on biological samples that have been previously collected from living animals (in particular human beings). In this case, the probes and the cleaving compounds of the invention are essentially used *in vitro,* without needing any interaction with the living animal.

Accordingly, the invention preferably targets the *in vitro* use of the probes and cleaving compound of the invention, or of the kit of the invention, to detect the presence of at least one biomarker molecule that is characteristic of a disease or disorder, in a biological sample, or to monitor the progression of a disease and/or the effect of a therapy, by measuring the level of one, preferably two, more preferably five or more (until 50, 75 or even 100) target biomarkers in biological samples of the subject with the system and methods of the invention at different time points, especially during said therapy.

The invention also targets an *in vitro* method, preferably an *in vitro* multiplex method, to detect in a biological sample the presence of at least one biomarker molecule that is characteristic of a disease or disorder, said method comprising the steps of:

a) Contacting said biological sample with an effective amount of the probes of the invention, *in vitro,* in appropriate conditions so that the probes of the invention can bind efficiently to the target biomarker molecule, if present in the biological sample,
b) Washing the sample (preferably with cold PBS) in order to remove the unbound antibodies,
c) After step b), contacting the biological sample with the cleaving compound of the invention, so as to release the MS-tags from the bound probes,
d) Detecting the nature and optionally the amount of the cleaved MS-tags released in step c) by mass spectrometry,

Wherein the nature and/or the amount of the cleaved MS-tags detected in step d) reveals the presence and / or the amount of the biomarker molecule in the biological sample.

The invention also targets *in vitro* multiplex methods according to the invention, in which the biological sample is contacted with at least two, preferably at least three, four, five or more (until 50, 75 or even 100) sets of probes as defined in the kit of the invention, so as to detect at least two, preferably at least three, four, five or more (until 50, 75 or even 100) different biomarker molecules of interest, with the same steps a) - d) defined above. In such multiplex methods, the nature and the amount of the biomarker molecules could be identified in one single measurement step.

This method can also be used to monitor the progression of a disease and/or the effect of a therapy, by measuring the level of one, preferably two, more preferably five or more (until 50, 75 or even 100) target biomarkers present in biological samples of the subject with the tools of the invention at different time points, especially during said therapy.

Of course, the nature and number of the probes of the invention should be appropriately selected so that they can bind efficiently to the (at least two) target biomarker(s) molecule(s). In other words, the number and nature of the probes of the invention should be adjusted to bind to as many biomarker molecules as required by the (multiplex) methods of the invention, as described above.

In particular, if the probe part of the probe of the invention is an antibody as proposed in the examples below, the "effective amount" and "appropriate conditions" of use of this probe should be adjusted to so as to detect efficiently the target biomarker protein(s). The antibody amount can be comprised for example between 0.1 and 1000 $\mu$g, preferably between 50 and 100 $\mu$g. The samples will generally be maintained at 4°C, but they can be kept at room temperature or they can be heated, as it is possible to use some antibodies at room temperature or even at 37°C. The contacting step a) can last for 1h to 12h, depending on the affinity of the antibody for its target molecule. All the necessary experimental conditions to be used will depend on the nature of the antibody or of the probe part and are available in the art.

If the cleaving compound is covalently coupled to a detectable label, this method may also contain an optional step of detecting the presence of said detectable label and/or measuring the amount of said detectable label in the biological sample, by using conventional means to detect these detectable labels.

Conventional means to detect the detectable labels are for example flow cytometry, FRET or BRET, single cell microscopic or histochemistry methods using single or multiple excitation wavelength and applying any of the adapted optical methods, such as electrochemical methods (voltammetry and amperometry techniques), atomic force microscopy, and radio frequency methods, e.g. multipolar resonance spectroscopy, confocal and non-confocal, detection of fluorescence, luminescence, chemiluminescence, absorbance, reflectance, transmittance, and birefringence or refractive index (e.g., surface plasmon resonance, ellipsometry, a resonant mirror method, a grating coupler waveguide method or interferometry), radioisotopic, magnetic resonance imaging, etc. In a preferred embodiment, the detectable label coupled to the cleaving compound of the invention is a fluorochrome and the means to detect its presence are flow cytometry or fluorescence microscopy.

The method of the invention may also contain a step of comparing the level of the cleaved MS-tags and/or of the detectable label present in the biological sample with at least one reference value. In this case, this reference value can be an absolute value, a relative value, a value that has an upper or a lower limit, a range of values, an average value, a median value, a mean value, or a value as compared to a particular control or baseline value. A reference value can be based on an individual sample value such as, for example, a value obtained from a sample from the subject being tested, but at an earlier point in time. The reference value can be based on a large number of samples, such as from population of subjects of the chronological age matched group, or based on a pool of samples including or excluding the sample to be tested. Preferably, the reference value used in the method of the invention is a mean value of the level of the biomarker of interest, obtained from reference samples collected in healthy subjects or from samples of the same patient collected at an earlier time point.

In the *in vitro* use / methods of the invention, the biological sample is possibly any sample that may contain at least one target biomarker molecule that is characteristic of a disease or disorder. Said biological sample can be for example a cell culture from an unknown origin or from a patient, a tissue sample collected by means of a biopsy or chirurgical operation, a cell lysate, or a urine, blood or plasma sample that has been collected from a human patient or from an animal.

In a preferred embodiment, said biological sample is a cell culture preferably obtained after a biopsy or from an excreta sample collected from a living animal.

*In vivo*

Alternatively, the probes of the invention and optionally the cleaving compounds described above can be administered *in vivo* to living animals (in particular to human beings) where they can bind to the target biomarkers *in situ,* and the amount of the detectable label and / or released MS-tags is thereafter measured *in vitro* on a biological sample collected from the living animal. These *in vivo* embodiments have been successfully tested by the inventors in the examples below with antibodies as probe parts. This immuno-profiling approach is likely to be more efficient than classic tumor biopsies immune-histochemical scoring as it relates the full complexity of the *in vivo* biology mechanisms.

Accordingly, the probes of the invention, and optionally the cleaving compounds of the invention, or the kit of the invention, as described above, are for use to detect at least one, preferably two, more preferably five or more (until 50, 75 or even 100) biomarker molecule(s) that is (are) characteristic of a disease or disorder in living animals or to monitor the progression of a disease and/or the effect of a therapy, by measuring the level of one, preferably two, more preferably five or more (until 50, 75 or even 100) target biomarkers in biological samples of the subject with the system and methods of the invention at different time points, especially during said therapy.

It is noteworthy that, when using the probes / kits of the invention, not all the detection steps require the presence of the living animal to be tested. Once the probes and the cleaving compound of the invention have been administered to the animal, then a biological sample is collected from the animal, and the last step of measuring the nature and the amount of the released MS-tags is performed on said biological sample, without requiring the presence of the animal any more. This last step is of technical nature, since it involves technical means to detect and quantify the presence of MS-tags in a biological sample.

More precisely, the invention targets in this case a method, preferably a multiplex method, for detecting at least one biomarker molecule that is characteristic of a disease or disorder in a living animal, said method comprising the steps of:

a) Administering to the living animal an effective amount of the probes of the invention as described above, so that the probes of the invention can bind to their target biomarker molecule, where and if present in the living animal,

b) Administering to the living animal the cleaving compound of the invention as described above, so as to release the MS-tags from the bound probes, if any,

c) Detecting, by mass spectrometry, the nature and optionally the amount of the released MS-tags in a biological sample collected from the living animal after step b),

wherein the nature and/or the amount of levels of MS-tags measured in step c) reveals the presence and / or the amount of the biomarker molecule present in said living animal.

The invention also targets multiplex methods in which the living animal is contacted with at least two, preferably at least three, four, five or more (until 50, 75 or even 100) sets of probes (the sets of probes being as defined above for the kit of the invention), so as to detect at least two, preferably at least three, four, five or more (until 50, 75 or even 100) different biomarker molecules of interest, with the same steps a) - c) defined above. In such multiplex methods, the nature and the amount of the biomarker molecules present in the living animal could be identified in one single measurement step.

This method can also be used to monitor the progression of a disease and/or the effect of a therapy, by measuring the level of one, preferably two, more preferably five or more (until 50, 75 or even 100) target biomarkers in biological samples of the subject with the tools of the invention at different time points, especially during said therapy.

Importantly, as explained above, step c) of these methods are performed *in vitro* by conventional mass spectrometry means, in biological samples that have been collected from the living animal. These samples can be collected without any harm for the patient / animal, especially when they are part of an excreta such as urine.

An alternative method according to the invention involves the following steps:

a) Administering to the living animal an effective amount of the probes of the invention as described above, so that the probes of the invention can bind to their target biomarker molecules, where and if present in the living animal,

b) Collecting a biological sample from said living animal, said biological sample containing the probes that are not bound to the biomarker molecules (if any),

c) Contacting said biological sample *in vitro* with the cleaving compound of the invention as described above, so as to release the MS-tags from the unbound probes, if present in the biological sample,

d) Detecting by mass spectrometry the nature and optionally the amount of the released MS-tags in the biological sample after step c),

wherein the nature and/or the amount of levels of MS-tags measured in step d) reveals the absence / presence and / or the amount of the biomarker molecules in said living animal.

In this method, steps c) and d) are performed *in vitro.*

In the methods of the invention involving administering the probes of the invention to the living animals, the biological sample collected afterwards is preferably an excreta sample such as plasma, blood, sperm, sweat or urine. More preferably, it is a urine sample harmlessly collected from the living animal.

Importantly, all the methods of the invention involve the technical step of analyzing the presence and / or quantity of a MS-tag in a biological sample collected from a living animal. This analyzing step is performed *in vitro* with conventional means of mass spectrometry.

Mass spectrometry (MS) is a powerful analytical technique used to measure the mass-to-charge ratio of ions. It is widely employed for determining masses of particles, identifying compounds within a sample, and elucidating the structure

and chemical properties of molecules. Various types of mass spectrometry technologies have been developed, each with its own unique applications and capabilities. Here are some of the mass spectrometry technologies available today: Time-of-Flight (TOF) MS, Quadrupole MS, Ion Trap MS, Linear Ion Trap (LIT) MS, Quadrupole Ion Trap (QIT) MS, Fourier Transform Ion Cyclotron Resonance (FT-ICR) MS, Triple Quadrupole MS (QqQ), Orbitrap MS, Sector MS, Magnetic Sector MS, Matrix-Assisted Laser Desorption/Ionization (MALDI) MS, Electrospray Ionization (ESI) MS, Atmospheric Pressure Chemical Ionization (APCI) MS, Thermal Ionization MS (TIMS), Secondary Ion MS (SIMS), Inductively Coupled Plasma MS (ICP-MS), Desorption Electrospray Ionization (DESI) MS, Direct Analysis in Real Time (DART) MS, Field Desorption (FD) and Field Ionization (FI) MS, Fast Atom Bombardment (FAB) MS. The choice of mass spectrometry technology depends on the specific application, the complexity of the sample, the range of molecule sizes expected, the need for quantification or structural analysis, and sensitivity and resolution requirements. Innovations and advancements in mass spectrometry continue to extend the capabilities and applications of these technologies. In the methods of the invention, the preferred MS technique used to detect the MS-tags released by the cleaving compound of the invention is tandem MS/MS, more preferably liquid chromatography tandem MS/MS (LC-MS/MS).

*Biomarkers*

The biomarker molecules according to the invention can be molecules (proteins, oligonucleotides, small chemicals, etc.) that are expressed only by cells present in animals suffering from a disease or disorder, and therefore not be expressed when the animal is healthy. In this case, the binding of the probes of the invention will help concluding that the animal is sick, whereas absence of binding will reveal that the animal is healthy.

The molecules proteins according to the invention may also be molecules (proteins, oligonucleotides, small chemicals, etc.) that are expressed only by healthy cells, and not by animal suffering from the disease or disorder. In this case, the binding of the probes of the invention will help concluding that the animal is healthy, whereas absence of binding will reveal that the living animal suffers from the disease / disorder.

Such biomarker molecules are well known in the art, especially for cancer. See e.g., in [17].

The methods / use of the invention may therefore use probes / antibodies specifically detecting any of these biomarkers.

The biomarker molecules detected by means of the tools developed by the present inventors are characteristic of a disease or disorder, and the methods and uses of the invention are of primary importance for diagnosing the onset of said disease or disorder, and / or their status.

As used herein, "diagnosing" or "identifying a subject having" refers to a process of identifying a disease, condition, or injury from its signs and symptoms. A diagnosis is notably a process of determining if an individual is afflicted with a disease or ailment (e.g., cancer). In a particular embodiment, the subject from which the sample is collected or on which the methods are performed has no symptoms and/or has not been diagnosed to suffer from a disease. Alternatively, the subject from which the sample is collected or on which the methods are performed may have symptoms and/or have been diagnosed to suffer from a particular disease, and the methods of the invention are used to confirm, precise, revise, or modify the first diagnostic. As used herein, a patient or animal "in need thereof" is a human or animal being suspected of suffering from a disease for any reason (age, symptoms, heredity, etc.) or a healthy human or animal (in which one want to detect a risk to suffer from a disease), or a human or animal that has already been diagnosed to suffer from a disease, said diagnostic requiring to be confirmed or revised.

In the context of the invention, the disease or disorder can be any disease or disorder whose diagnosis can depend on the presence or absence of at least one biomarker molecule. While not all diseases can be conclusively diagnosed solely on cellular biomarkers, many conditions have associated biomarkers that aid in diagnosis, prognosis, and monitoring of disease. Here's a list of some diseases and conditions that are frequently associated with specific cellular biomarkers:

- Cancer (Breast cancer (HER2 & HER3, ER/PR); Prostate cancer (PSA); Colorectal cancer (CEA, KRAS mutations); Lung cancer (EGFR mutations, ALK rearrangements); Leukemia (BCR-ABL fusion protein, CD markers); Lymphoma (CD markers, BCL-2); Melanoma (BRAF mutations); Ovarian cancer (CA-125); Pancreatic cancer (CA 19-9); Thyroid cancer (Thyroglobulin); Other cancer biomarkers being listed on the ACCC website: https://www.accc-cancer.org/home/learn/precision-medicine/cancer-diagnostics/biomarkers/biomarkerlive/lexicon/cancer-biomarkers#P;
- Cardiovascular Diseases (Acute myocardial infarction (Cardiac troponins, CK-MB); Heart failure (BNP, NT-proBNP)
- Neurological Disorders (Alzheimer's disease (Amyloid beta, Tau proteins); Parkinson's disease ($\alpha$-Synuclein); Multiple sclerosis (Neurofilament light chain, Oligoclonal bands)
- Metabolic Disorders (Diabetes mellitus (Glucose, Hemoglobin A1c, C-peptide); Dyslipidemias (LDL, HDL, triglycerides))
- Inflammatory and Autoimmune Diseases (Rheumatoid arthritis (Rheumatoid factor, anti-CCP antibodies); Systemic lupus erythematosus (ANA, anti-Smith antibodies); Inflammatory bowel disease (Fecal calprotectin, CRP); Celiac

disease (Anti-tissue transglutaminase antibodies).

The methods of the invention may therefore use probes and/or antibodies specifically detecting any of these biomarkers.

*Cancer*

In a particular embodiment, the method / use of the invention is performed to detect at least one biomarker molecule that is present at the surface of cancer cells. Preferably, the biological sample is thus a cancer cell culture, a tumor tissue collected by means of a biopsy, or a fluid sample collected from a human or animal subject suffering from cancer.

Cancer biomarkers are biological molecules found in blood, other body fluids, or tissues that can be associated with the presence of cancer, providing information about the likely course of the disease, and response to treatment. A number of cancer biomarkers are currently used to screen, diagnose, prognose, and/or monitor a treatment response ([17]).

In a particular embodiment, the methods and use of the invention enable to diagnose that the living animal from which the biological sample originates suffers from cancer.

More particularly, the invention targets an *in vitro* method, preferably a multiplex *in vitro* method, for diagnosing a cancer in a patient in need thereof by detecting at least one biomarker molecule present in a biological sample of said patient, said method comprising the steps of:

a) Contacting said biological sample with at least one set of the probes of the invention, these probes specifically targeting at least one biomarker molecule specifically expressed by or absent in cancer cells, so that the probes of the invention can bind to said target biomarker molecule, where and if present in said sample,
b) Optionally washing the sample in order to remove the unbound probes,
c) Contacting the biological sample with the cleaving compound of the invention, so as to release the MS-tags coupled to the bound probes,
d) Measuring the levels of the released MS-tags by mass spectrometry, preferably by LC-MS/MS.

The invention also targets *in vitro* diagnosis multiplex methods according to the invention, in which the biological sample is contacted with at least two, preferably at least three, four, five or more (until 50, 75 or even 100) sets of probes as defined above for the kit of the invention, so as to detect the presence in the sample of at least two, preferably at least three, four, five or more (until 50, 75 or even 100) different biomarker molecules of interest, with the same steps a) - d) defined above.

In this method, the nature and the amount of each MS-tag released in step d) will indicate which biomarker molecule is present or absent in the biological sample in one single analysis. Depending on the biomarker at stake, this method will enable to conclude the diagnosis of the cancer from which the patient is likely to suffer, or monitor the evolution of the disease, in one single analysis.

Optionally, and as explained above, it will also be possible to analyze the localization of the biomarker molecule on the cells present in the sample (if any), if the cleaving compound is labelled with a detectable label.

In another embodiment, the invention targets a method, preferably a multiplex method, for diagnosing a cancer in a patient in need thereof, said method comprising the steps of:

a) Administering to said patient an effective amount of at least one set of probes as described above, these probes specifically targeting at least one biomarker molecule specifically expressed by or absent in cancer cells, so that the probes of the invention can bind to said target biomarker molecule, where and if present in said patient,
b) Administering to the patient the cleaving compound of the invention as described above, so as to release the MS-tags from the bound probes, if any,
c) Detecting by mass spectrometry the nature and/or the amount of the released MS-tags in a biological sample (preferably an excreta sample) collected from the patient after step b),

wherein the nature and/or the amount of levels of MS-tags measured in step c) reveals the presence and / or the amount of the biomarker molecule present in said patient and therefore permits cancer diagnosis.

The invention also targets diagnosis multiplex methods according to the invention, in which the patient is contacted with at least two, preferably at least three, four, five or more (until 50, 75 or even 100) sets of probes as defined above for the kit of the invention, so as to detect at least two, preferably at least three, four, five or more (until 50, 75 or even 100) different biomarker molecules of interest, with the same steps a) - c) defined above.

In these *in vivo* methods, the administration of steps a) and b) can be systemic (e.g., intraperitoneally or intravenously) or, if the patient suffers from a solid cancer that is already detected, steps a) and b) can be performed

intratumorally. The administrations of steps a) and b) can also be done by different routes, for example the administration of step a) can be performed systemically, whereas the administration step b) can be performed intratumorally.

The timing between the administration of the probes of the invention (step a) and the administration of the cleaving compound of the invention (step b) should be adjusted so that all the probes can reach and efficiently binds to the target biomarker molecules, if present. It is typically between 2 and 3 days in mice, and would be for example comprised between one and 5 days in human beings, for full-length antibodies.

The timing between the administration of the probes of the cleaving compound of the invention (step b) of the multiplex *in vivo* method of the invention) and the collection of the biological sample (step c) of the multiplex *in vivo* method of the invention) should be adjusted so that all the cleaving compound can reach and efficiently binds to the probes of the invention, where bound, and all the MS-tags released in the excreta sample so that the MS-analysis reflects the actual amount of the target biomarker molecules. It is typically of one day in mice, and would be for example comprised between one and 5 days in human beings, for full-length antibodies.

If the patient is likely to suffer from a liquid cancer (or if no solid cancer has been detected in the patient yet), the administration of steps a) and b) can be both systemic (e.g., intraperitoneally or intravenously).

In another embodiment, the invention targets a multiplex method for diagnosing a cancer in a patient in need thereof, said method comprising the steps of:

a) Administering to the patient an effective amount of the probes of the invention as described above, so that the probes of the invention can bind to the target biomarker molecules, where and if present in the patient,
b) Collecting a biological sample from said patient, said biological sample being preferably an excreta sample containing the probes that are not bound to the biomarker molecules (if any),
c) Contacting said biological sample *in vitro* with the cleaving compound of the invention as described above, so as to release the MS-tags from the unbound probes, if present in the biological sample,
d) Detecting the nature and optionally the amount of the released MS-tags in the biological sample after step c), by mass spectrometry,

wherein the nature and/or the amount of levels of MS-tags measured in step d) reveals the presence and / or the amount of the biomarker molecules present in said patient and therefore permits cancer diagnosis.

The timing between the administration of the probes of the invention (step a) and the collection of the biological sample (step b) should be adjusted so that all the probes can reach and efficiently binds to the target biomarker molecules, if present and, in the contrary case, reach the systemic system to be excreted. It is typically of 3 days in mice, and would be for example comprised between one and 5 days in human beings, for full-length antibodies.

The methods and use of the invention are in particular useful to :

- Detect or diagnose a tumor in a patient in need thereof,
- Characterize the biomarkers expressed by the tumor when it has been detected,
- Select the more appropriate anti-cancer therapy according to the tumor characterization,
- Monitor the progression of the tumor before or after a treatment is initiated, or to
- Monitor the effect of an anti-cancer treatment,

by measuring the level of the biomarkers in biological samples of the subject with the system and methods of the invention at different time points, especially during treatment.

It is always possible to add in any of the methods of the invention a confirmation step involving analyzing a biopsy tissue of the tumor, by conventional *in vitro* means (e.g., immunohistochemistry, western blot, etc.).

Nevertheless, the advantage of the methods of the invention is that they enable to characterize and/or diagnose and/or monitor tumors in their native environment without the need of any tissue removal.

Screening methods of the invention

In a fourth aspect, the present invention also targets the use of the probes and cleaving compound of the invention, or of the kit of the invention, for identifying new antibodies or new biological markers that can serve in immunotherapeutic treatments.

For example, the present invention targets an *in vitro* method to screen and identify probes that can be efficient for immunotherapy or as chemotherapy drug carriers, said method comprising the following steps:

a) Contacting a sample comprising tumor cells with an effective amount of candidate probes that have been processed according to the invention, in appropriate conditions so that the candidate antibodies can bind efficiently to the target biomarker molecules, if present in the biological sample,

b) Contacting a sample comprising healthy cells with the same effective amount of the same candidate probes, in the same appropriate conditions as in step a),

c) Washing the samples in order to remove the unbound probes,

d) After step c), contacting the samples with the cleaving compound of the invention, so as to release the MS-tags from the bound probes, if any,

e) Detecting the nature and optionally the amount of the cleaved MS-tags released in step d) by mass spectrometry, in the two samples,

wherein the nature and/or the amount of the cleaved MS-tags detected in step e) reveals the presence and / or the amount of the biomarker molecules specifically in the sample containing tumor cells and therefore the probes that detect cancer cells specifically.

In particular, the present invention targets an *in vitro* method to screen and identify antibodies that can be efficient for immunotherapy or as chemotherapy drug carriers, said method comprising the following steps:

a) Contacting a sample comprising tumor cells with an effective amount of candidate antibodies that have been processed according to the invention, in appropriate conditions so that the candidate antibodies can bind efficiently to the target biomarker proteins, if present in the biological sample,

b) Contacting a sample comprising healthy cells with the same effective amount of the same candidate antibodies, in the same appropriate conditions as in step a),

c) Washing the samples in order to remove the unbound antibodies,

d) After step c), contacting the samples with the cleaving compound of the invention, so as to release the MS-tags from the bound antibodies, if any,

e) Detecting the nature and optionally the amount of the cleaved MS-tags released in step d) by mass spectrometry, in the two samples,

wherein the nature and/or the amount of the cleaved MS-tags detected in step e) reveals the presence and / or the amount of the biomarker proteins specifically in the sample containing tumor cells and therefore the antibodies that detect cancer cells specifically.

The candidate antibodies binding only to the cells of the sample of step a) will target biomarker proteins that are present at the surface of cancer cells, and they can therefore be used to carry chemotherapeutic drugs in antibody-drug-conjugates (ADCs), vectorized internal radiotherapy or in other immunotherapeutic treatments.

Additionally, the system of the invention could be also used to identify new drugs that would be effective in treating diseases or disorders associated with a specific biomarker, for example by comparing the effect of a candidate drug on the expression of said biological marker in several conditions (comparison between normal / abnormal cells, between markers of normal / abnormal cells, before or after the treatment, etc.).

All the details concerning the antibodies, cleaving compounds, MS techniques, detectable labels, timing and conditions are the same as described above for the *in vitro* method of the invention described above, and need not be repeated here.

## Definitions

In the context of this description, a halogen atom is a chlorine, iodine, bromine or fluorine atom. Preferably, a halogen atom is a bromine or a chlorine atom, in particular a bromine atom. An alkyl group is a linear saturated hydrocarbon group comprising from 1 to 20 carbon atoms, a branched saturated hydrocarbon group comprising from 3 to 20 carbon atoms or a cyclic saturated hydrocarbon group comprising from 4 to 20 carbon atoms. Preferably, the alkyl group according to the invention comprises from 1 to 10, in particular from 1 to 6, carbon atoms. Examples of alkyl groups comprise methyl, ethyl, propyl, isopropyl, butyl, tertbutyl, isobutyl, n-pentyl, n-hexyl and cyclohexyl groups. An alkenyl group (or an alkene) is a linear hydrocarbon group comprising from 2 to 20 carbon atoms, a branched hydrocarbon group comprising from 4 to 20 carbon atoms, or a cyclic hydrocarbon group comprising from 4 to 20 carbon atoms and comprising at least one C=C double bond. Preferably, the alkenyl group according to the invention comprises from 2 to 10, in particular from 2 to 6, carbon atoms. Examples of alkenyl groups comprise ethylenyl, propylenyl and cyclohexenyl groups.

An alkynyl group (or an alkyne) is a linear hydrocarbon group comprising from 2 to 20 carbon atoms, a branched hydrocarbon group comprising from 4 to 20 carbon atoms or a cyclic hydrocarbon group comprising from 4 to 20 carbon atoms and comprising at least one C≡C triple bond. Preferably, the alkynyl group according to the invention comprises from 2 to 10, in particular from 2 to 9, carbon atoms. Examples of alkynyl groups comprise ethynyl, propynyl, octynyl, cyclooctynyl and cyclononynyl groups. In specific embodiments of the present invention, the alkyl, alkenyl and/or alkynyl groups may be interrupted by at least one heteroatom, preferably independently selected from nitrogen, oxygen and sulphur atoms.

An alkoxy group is an alkyl group, bonded to the rest of the molecule through an oxygen atom. An aralkyloxy group is an aralkyl group, such as a benzyl group, bonded to the rest of the molecule through an oxygen atom.

A thioether group is an alkyl group, bonded to the rest of the molecule through a sulfur atom. An aryl diazo group is a $N_2$ group bonded to an aromatic group. A carboxyl group is a COOH group. A nitro group is a $NO_2$ group.

An amino group is a $NR_1R_2$ group, wherein $R_1$ and $R_2$ are independently selected from the group consisting of hydrogen atoms, aromatic groups (such as a phenyl or tolyl group) and alkyl groups. In an embodiment, at least one of $R_1$ and $R_2$ is an alkyl group. In an embodiment, such an amino group is an alkylamino group. In an embodiment, $R_1$ and $R_2$ are both alkyl groups. In an embodiment, such an amino group is a dialkylamino group.

An acyl group is a functional group obtained by removing a hydroxyl group (OH) from the acid's carboxyl group (-COOH). An acyl group has the general formula R-C(=O)-, where R can be an alkyl, alkenyl, aryl, or any other organic group, and the C=O represents the carbonyl group.

A sulfonyl group is a functional group characterized by a sulfur atom doubly bonded to two oxygen atoms and single bonded to an organic moiety, usually represented as $R\text{-}S(=O)_2$, where R represents any organic group such as an alkyl, aryl, or other carbon-containing group attached to the sulfur.

An "activated ester" is an ester with a good leaving group, such as a N-hydroxysuccinimide ester, a N-hydrophthalimide ester, a perfluorinated ester or an acylurea.

In the context of the present invention, the term **"biomarker"** relates to a naturally occurring molecule, preferably a biomolecule, gene, or molecular group by which a particular pathological or physiological process, disease, etc. can be identified.

By **"detecting",** it is herein meant that the antibodies or kits of the invention enable to localize the biomarker protein, to specifically identify their presence or their absence in the sample, and optionally to quantify the amount of the protein in the sample. As a matter of fact, localization of the protein can be done when the cleaving compound is coupled to a detectable label as described above, and quantification can be performed by any conventional means, either by measuring the amount of the detectable labels that are specifically bound to the cells (by fluorescence microscopy, radioisotopic devices, etc.) or by measuring the amount of the released MS-tags by mass spectrometry once the cleaving compound has been contacted.

By **"living animal",** it is herein included any animal (including human beings) that can be diagnose with a disease or disorder, especially with cancer. It can also be a farm or a pet animal, such as duck, chicken, cat, dog, mice, rat, horse, rabbit, goat, sheep, cow, etc.

## FIGURE LEGENDS

**Figure 1** proposes different strategies for multiplexed cancer cell profiling : **(A)** method of the prior art, involving using several fluorescently-labelled antibodies whose fluorophores need to be inactivated before another cycle is iterated, **(B)** the method of the invention, using the MS-tagged antibodies of the invention, whose MS-tags can be removed easily without deleterious treatments of the target cells.

**Figure 2** discloses the structure of the antibodies of the invention, and shows the biorthogonal cleavage reaction onto the iminosydnone cleavable linker linking the antibody to the tags when the alkynyl cleaving compound DBCO is added, releasing the MS-tag linked to antibody.

**Figure 3** shows the synthesis pathway of TMPP isotopologue compounds, TMPP-H24, TMPP-H15 and TMPP-H6 starting from the commercially available TMPP-H33 compound.

**Figure 4** discloses the synthesis pathway of the MS-tags tris(2,4,6,-trimethoxyphenyl)-phosphonium (TMPPs) compounds coupled to a cleavable linker, which is in this case the compound 4, i.e., a iminosydnone compound containing PEG spacers, whose alcohol function was then tosylated to allow reaction with trimethoxybenzene phosphine and three of its deuterated analogues to obtain four phosphoniums 5 bearing TMPP isotopologues: TMPP-H33, H24, H15 and H6.

**Figure 5** shows (A) the synthesis pathway of the antibodies of the invention, using the TMPP isotopologue compounds of figure 4 and (B) the release of the TMPP tags following DBCO treatment.

**Figure 6** shows the fluorescent evaluation of the specific and unspecific binding of CTX-H24 and DBCO-TAMRA probes by incubated A431 cells with or without excess of CTX in at 37°C. Results are represented as mean ± standard deviation (n = 3, 750 000 cells).

**Figure 7** shows a binding assay using mass spectrometry analysis. **A)** Concentrations of released tags from left to right: TRZ-H33 (0.8 mg.mL$^{-1}$), CTX-H24 (1.06 mg.mL$^{-1}$), DUR-H15 (1.281 mg.mL$^{-1}$) and BVZ-H6 (0.54 mg.mL$^{-1}$) associated to the H1975, A431, U87 and SkBr3 cells, respectively with or without an excess of themselves (100 x). Results are represented as mean $\pm$ standard deviation (n = 3, 750 000 cells). **B)** In the saturation binding assay using CTX-H24, the $B_{max}$ and $K_d$ values were determined through specific binding with hill slope analysis from GraphPad. These results were then compared under identical conditions with CTX conjugated with fluorescein (FITC). C) FITC was mixed with CTX and incubated at room temperature for 60 minutes to achieve covalent conjugation. Unreacted FITC was subsequently removed using a PD-10 column. The FITC-to-antibody ratio was assessed through fluorescence measurement (BMG, Clariostar), yielding a ratio of 2.13. Subsequently, CTX-FITC was incubated at various concentrations in A431 cells, mirroring the conditions used in B). Non-specific binding was subtracted from the total binding, resulting in the specific binding presented in nM. The $K_d$ values for both CTX-FITC and CTX-H24 were found to be in the same range, approximately 10 to 20 nM, indicating a minimal impact of H24 conjugation on CTX binding.

**Figure 8** displays the proof of concept of the click and release approach combining bioorthogonal click labeling and fluorescence imaging with tag release and LC-MS/MS-analysis. **A)** Reagent structures and schematic principle of the technology; **B)** Fluorescent binding assay to evaluate the specific versus non-specific binding of CTX-H24 and DBCO-TAMRA. A431 cells were incubated with or without excess of CTX at 37 °C or 4 °C; C) LC-MS/MS quantification of released TMPP-H24 tag after binding of CTX-H24 on A431 cells and subsequent treatment with TAMRA-DBCO with or without excess of CTX (2 h, 37°C).

**Figure 9** shows the multiplexed LC-MS/MS detection of cell membrane receptors on tissue slices, using our click and release approach. A) Principle of the multiplex detection. B) Fluorescence imaging of biomarkers after addition of DBCO-TAMRA in absence or presence of antibodies. C) Quantification of biomarkers using LC-MS/MS.

**Figure 10** shows the *in vivo* immunoprofiling using the click-and-release approach of the invention. **A)** Principle of the experimental *in vivo* procedure. **B)** Biodistribution of [$^{18}$F]-DBCO 6 determined by PET imaging (p.i.: post-intratumoral injection). **C)** Ex vivo analysis of TMPP-tags released in urines and remaining in the tumor after intratumoral injection of [$^{19}$F]-6. Concentration values were normalized in function of the TAR. $H_{24}$ and $H_{15}$ in urine and in the injected tumor was significantly higher than the H33 and H6 (p < 0.01, Kruskal-Wallis test).

**Figure 11** shows the pharmacokinetic data of TMPP-Tags after injection of the 4 Tags. Blood sampling was performed at different time points from 30 min to 2 hours. Urine was sampling during 24 h. Twenty-four hours after injection, the mice were euthanized and the organs of interest (blood, kidneys, liver and tumors) were extracted for MS analysis.

**Figure 12** shows the preparation and characterization of tagged mAbs and the TMPP tag release from antibodies upon SPSIC reaction with DBCO. TAR = Tag-Antibody-Ratio.

## EXAMPLES

### Material and methods

All chemical products commercially available were purchased from Sigma-Aldrich, Acros and Fluka and used without further purification. Anhydrous solvents: were purchased in anhydrous form and used without further purification. Dichloromethane was distilled form calcium hydride under nitrogen. Tris(2,4,6-trimethoxyphenyl)phosphine was commercial available and was purchased from Acros. Reactions were monitored by TLC carried out on silica 0,25 mm (60 F254, Merck) using UV light as visualizing agent and basic aqueous permanganate as developing agent. $^{1}$H NMR (400 MHz), $^{13}$C NMR (100 MHz) were measured on a Brucker Advance 400 MHz spectrometer. Chemical shifts are reported in parts per million (ppm) downfield from residual solvents peaks and coupling constants are reported as Hertz (Hz). Splitting patterns are designated as singlet (s), broad singlet (br. s), doublet (d), triplet (t), quartet (q), quintet (quint), heptuplet (hept), multiplet (m). Splitting patterns that could not be interpreted or easily visualized are designated as multiplet (m). LCMS mass spectra were recorded using a single quadrupole mass spectrometer (SQD 2, Waters) with electrospray source coupled to Ultra-High Performance Liquid Chromatography (Acquity UPLC H-Class, Waters). High resolution mass spectroscopy of the final compounds were determined using a Xevo® G2-XS QT of Infrared spectra (IR) were obtained on a Perkin Elmer system 2000 FT-IR spectrophotometer or a Perkin Elmer UATR TWO FTIR spectrophotometer and are reported as wavelength numbers (cm-1). Melting points (Mp) were obtained on a BÜCHI Melting Point B-545 and are reported in °C. Absorbances were measured on a UV JASCO V-750 equipped with an injection module (GSP-909) and a Peltier (EHCS-760).

Synthesis of tris-trimethoxyphenylphosphines (TMP) compounds (figure 4):

**TMP-H33** is commercially available. The other TMPs were synthesized in 2 steps according to Figure 4.

**General procedure for trimethoxybenzene (TMB) synthesis**

The appropriate phenol (1.0 equiv.) was suspended in dry acetone (0.5 M) in an oven dry sealed tube, then $K_2CO_3$ and deuterated-dimethylsulfate (1.1 equiv./deuterable position) were successively added to the solution. The resulting mixture was then heated at 60 °C for six hours. After completion of the reaction, the crude product was evaporated and purified by column chromatography on silica gel (Heptane/AcOEt 90/10) to give the desired trimethoxybenzene :

- 1,3-dimethoxy-5-(methoxy-d3)benzene - TMB-D3

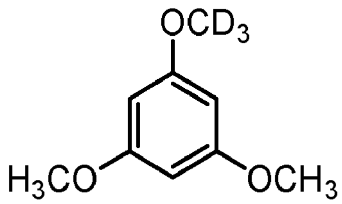

- 1-methoxy-3,5-bis(methoxy-d3)benzene - TMB-D6

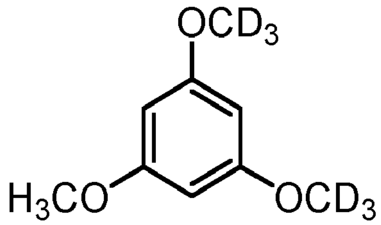

- 1,3,5-tris(methoxy-$d_3$)benzene - TMB- D9

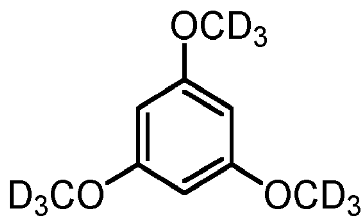

**General procedure fortris-trimethoxyphenylphosphines TMP synthesis**

Trimethoxybenzene (3.0 equiv.) and $ZnCl_2$ extra dry (1.0 equiv. stored in glovebox) were added into a sealed vial in glovebox. The vial was sealed, then $PCl_3$ (3.0 equiv.) was added. The reaction mixture was stirred at 80 °C for 16 h, then cooled to room temperature. Toluene (2.0 mL) was added dropwise very carefully to the mixture at 0 °C and then removed to afford a viscous residual complex of $ZnCl_2$. Aqueous ammonia was then added at 0 °C and the solution was extracted with toluene. The organic phases were combined, dried over $MgSO_4$ and evaporated under reduced pressure. The crude material was recrystallized in $Et_2O$ to afford the desired phosphine.

- tris(2,6-dimethoxy-4-(methoxy-d3)phenyl)phosphine - TMP-H24

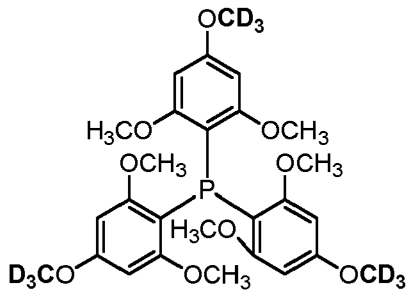

- tris(6-methoxy-2,4-bis(methoxy-d3)phenyl)phosphine - TMP-H15

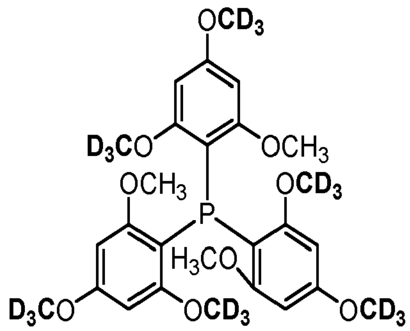

- tris(2,4,6-tris(methoxy-d3)phenyl)phosphine - TMP-H6

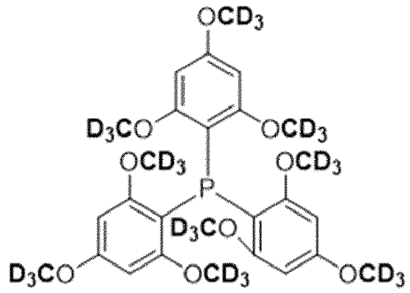

This compound was obtained as described in the literature [24].

Synthesis of the iminosydnone linker (see figure 3):

(3-(4-(ethoxycarbonyl)phenyl)-1,2,3-oxadiazol-3-ium-5-yl)(1H-imidazole-1-carbonyl)amide = compound 1 of formula :

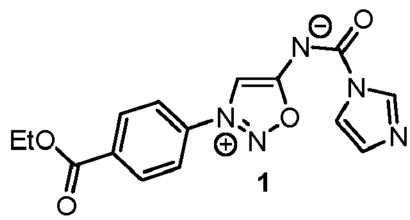

**1**

This compound was obtained as previously described by [19].

Compound 1 (2.0 g, 6.1 mmol, 1.0 equiv.) was dissolved in a mixture of THF (7.5 mL) and $H_2O$ (7.5 mL). NaOH (0.269 g, 6.7 mmol, 1.1 equiv.) in water (1.0 mL) was added and the reaction mixture was stirred at room temperature for 2 h. THF was then evaporated, and the water solution was washed with DCM. Then, HCl (1 M) was added until pH reached 3 and the precipitate was collected by centrifugation and dried under vacuum. The yellow wish solid was solubilized in a mixture of MeCN (7.5 mL) and DMF (7.5 mL) and DiPEA (3.1 mL, 18.3 mmol, 3.0 equiv.) was added followed by HATU (2.8

g, 7.3 mmol, 1.2 equiv.) and $NH_2PEG_3CO_2{}^tBu$ (1.56 g, 6.7 mmol, 1.1 equiv.). Solvents were removed under vacuum and the crude mixture was purified by column chromatography (DCM 100 % to DCM/MeOH 94/06) to afford the compound **2** as a yellow moss (1.3 g, 2.5 mmol, 42 % over two steps).

(3-(4-((2-(2-(3-(tert-butoxy)-3-oxopropoxy)ethoxy)ethyl)carbamoyl)phenyl)-1,2,3-oxadiazol-3-ium-5-yl)(1H-imidazole-1-carbonyl)amide = compound **2** of formula:

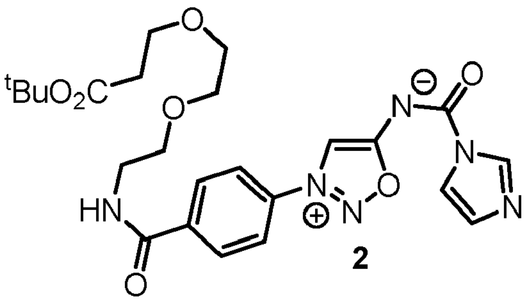

**2**

Compound 2 (0.800 g, 1.55 mmol, 1.0 equiv.) was dissolved in MeCN (8.0 mL). MeI (0.387 mL, 6.22 mmol, 4.0 equiv.) was added and the reaction mixture was stirred at room temperature for 16 h. Then, volatiles were removed and $CHCl_3$ (stabilized on amylene, 15.0 mL) and $NH_2PEG_4CO_2{}^tBu$ (0.330 g, 1.70 mmol, 1.1 equiv.) were successively added and stirred at room temperature for 16 h. Solvent was removed under vacuum and the crude mixture was purified by column chromatography (DCM/MeOH 94/06) to afford the compound **3** as a yellow oil (0.582 g, 0.910 mmol, 52 % over two steps).

(3-(4-((2-(2-(3-(tert-butoxy)-3-oxopropoxy)ethoxy)ethyl)carbamoyl)phenyl)-1,2,3-oxadiazol-3-ium-5-yl)((2-(2-(2-(2-hydroxyethoxy)ethoxy)ethoxy)ethyl)carbamoyl)amide = compound 3 of formula :

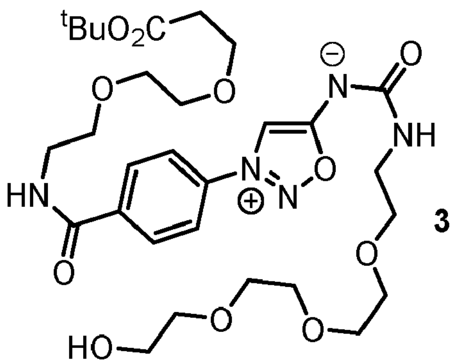

**3**

Synthesis of TMPP tagged linkers = compound 5 (figure 3):

$Et_3N$ (0.527 mL, 3.91 mmol, 5.0 equiv.) and TsCl (0.297 g, 1.56 mmol, 2.0 equiv.) were added to a solution of **3** (0.500 mg, 0.78 mmol, 1.0 equiv.) in dry DCM (50.0 mL). The mixture was stirred overnight at room temperature. DCM was then removed under reduced pressure and the crude mixture was rapidly purified by flash chromatography DCM/MeOH 98/02, divided in 4 batches, and directly engaged in the next step. The tosylated intermediate (1.0 equiv.) was dissolved in dry DMF (0.2 M) and then $P(Ph(OMe)_3)_3$ (1.0 equiv.) and NaI (1.1 equiv.) were added to the solution and the mixture was heated overnight at 40 °C. Then, DMF was removed under reduced pressure and DCM 4.0 mL was added to precipitated impurities. The suspension was centrifuged and the supernatant was collected and evaporated under reduced pressure. The crude mixture was finally purified by column chromatography on silica gel (slowly DCM 100 % to DCM/MeOH 95/05) to give the desired product **5** as a yellow oil.

((2-(2-(2-(2-(bis(4-((l1-oxidaneyl)-l5-methyl)-2,6-dimethoxyphenyl)(2-((l1-oxidaneyl)-l5-methyl)-4,6-dimethoxyphenyl)phosphonio)ethoxy)ethoxy)ethoxy)ethyl)carbamoyl)(3-(4-((2-(2-(3-(tert-butoxy)-3-oxopropoxy)ethoxy)ethyl)carbamoyl)phenyl)-1,2,3-oxadiazol-3-ium-5-yl)amide iodide - **5-H$_{33}$**

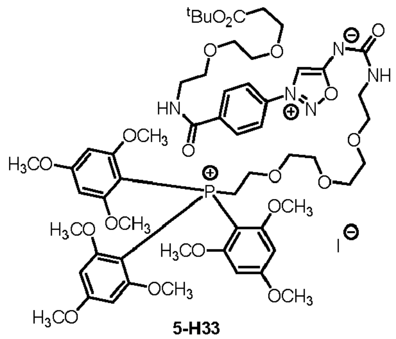

5-H33

((2-(2-(2-(2-((2-((l1-oxidaneyl)-l5-methyl)-6-methoxy-4-(methoxy-d3)phenyl)bis(2,6-dimethoxy-4-(methoxy-d3)phenyl)phosphonio)ethoxy)ethoxy)ethoxy)ethyl)carbamoyl)(3-(4-((2-(2-(3-(tert-butoxy)-3-oxopropoxy)ethoxy)ethyl)carbamoyl)phenyl)-1,2,3-oxadiazol-3-ium-5-yl)amide iodide - **5-H$_{24}$**

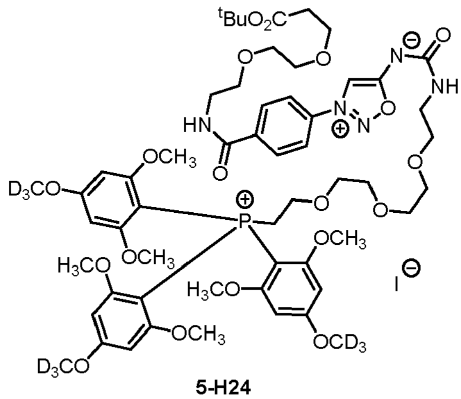

5-H24

((2-(2-(2-(2-(bis(4-((l1-oxidaneyl)-l5-methyl)-2,6-bis(methoxy-d3)phenyl)(4-methoxy-2,6-bis(methoxy-d3)phenyl)phosphonio)ethoxy)ethoxy)ethoxy)ethyl)carbamoyl)(3-(4-((2-(2-(3-(tert-butoxy)-3-oxopropoxy)ethoxy)ethyl)carbamoyl)phenyl)-1,2,3-oxadiazol-3-ium-5-yl)amide iodide - **5-H$_{15}$**

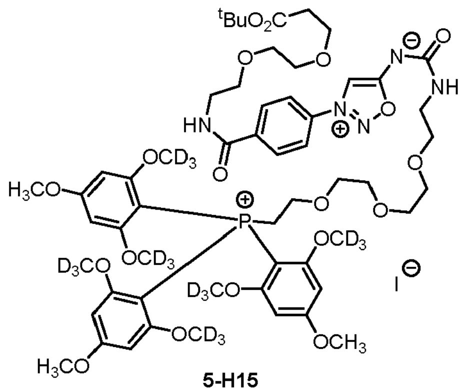

5-H15

(3-(4-((2-(2-(3-(tert-butoxy)-3-oxopropoxy)ethoxy)ethyl)carbamoyl)phenyl)-1,2,3-oxadiazol-3-ium-5-yl) ((2-(2-(2-(2-(tris(2,4,6-tris(methoxy-d3)phenyl)phosphonio)ethoxy)ethoxy)ethoxy)ethyl)carbamoyl)amide iodide - **5-H$_6$**

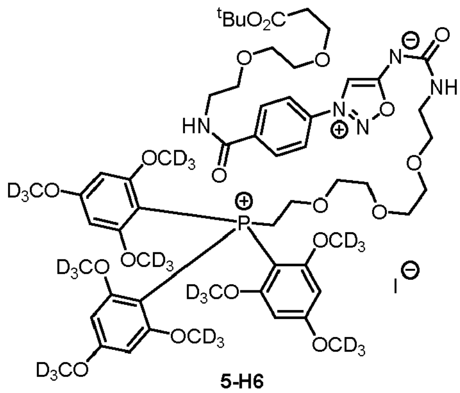

**5-H6**

TMPP-antibody bioconjuqation (figure 5A):

The compound 5 was dissolved in a solution of DCM/TFA 1/1 (0.05 M) and the solution was stirred for 2 hours. After completion of the reaction, the crude mixture was evaporated under high vacuum to give the corresponding carboxylic acid quantitatively. To this acid (1.0 equiv.), TSTU (1.0 equiv.) and Et3N (1.0 equiv.) were added in dry DMF and stirred at 25 °C for 3 hours. Then 5-10 μL, depending of the mAb, of this solution (10-20 equiv.) were then added to 1.0 mL of a solution of mAb at 2.0 mg/mL in 0.1 M NaHCO3 pH 8.3. The mixture was then incubated at 25°C for 4 hours and the resulting mAbs-Hxx were purified via a minitrap® cartridge (1000 μL loading, 1000 μL elution of 0.9 % NaCl in DI water).

Table 1. Preparation and characterization of TMPP-mAbs

| TMPP-mAb | 5-Hxx (equiv.) | Concentration (BSA assay) | TAR |
|---|---|---|---|
| **TRZ-H33** | **5**-H33 (20 equiv.) | 1,26 mg/mL | 4.16 |
| **CTX-H24** | **5**-H24 (20 equiv.) | 1.06 mg/mL | 2.49 |
| **DUR-H15** | **5**-H15 (10 equiv.) | 1.28 mg/mL | 2.35 |
| **BVZ-H6** | **5**-H60 (20 equiv.) | 0.54 mg/mL | 3.44 |

BCA assay :

The concentration of antibodies functionalized with TMPP tags was determined by BCA assay. Briefly in a 96-well plate, 90 μL of the BCA Working Reagent solution were added to 10 μL of the antibody solution and incubated 30 min at 37 °C. After 5 min, the temperature was cooled down to room temperature; the absorbance of the solution at 562 nm was measured on a microplate spectrophotometer (CLARIO Start Plus, BM6 LABTECH). The antibody concentration was determined by comparison to a standard calibration curved made with BSA protein at concentration ranging from 0.2 to 1 g/L.

TAR (Tag-Antibody-Ratio) determination (figure 5A and figure 12):

Due to the permanent positive charge of TMPP tags, the antibody-conjugates are multicharged making impossible a direct analysis of the conjugates by MALDI. The determination of TAR was therefore carried out by analyzing the amount of released TMPP tags after click and release SPSIC reaction with DBCO.

DBCO (10 μL, 100 μM, 100 equiv./mAbs) was added to a solution of mAbs-Hxx (100 μL, 1 equiv.) in 0.9 % NaCl solution. The mixture was agitated overnight, and the released Tag was quantified in this solution by HPLC. Knowing the concentration of both the antibody and the tag, the TAR was calculated by dividing the amount the TAG-Hxx by the amount of antibody.

Kinetic studies with TMPP-mAbs conjugates:

Reactions were followed by measuring the increase of concentration of the released TMPP tags by measuring the apparition of fragment ions Frag-H6, 15, 24 and 33 by LC-HRMS/MS.

5 μL of TMPP-mAbs conjugate solutions (TRZ-H33 8,4 μM, CTX-H24 7,1 μM, DUR-H15 8,5 μM, BVZ-H6 5,6 μM) were diluted in 1485 μL of PBS 0.1 M. The solution was equilibrated at 37 °C in the LC autosampler before adding 10 μL of DBCO-Acid (50 equiv. in HPLC grade DMSO). To ensure selective measurement of the TMPP tag concentrations, LC-MS/MS analysis were performed. TMPP-H6, 15, 24 and 33 were selected as precursor ions and signals of fragment ions Frag-H6, 15, 24 and 33 were detected. 5 μL of the reaction mixture were regularly injected in the LC-MS instrument to follow the reaction. The peak of fragments Frag-H6, 15, 24 and 33 were integrated in the chromatograms. Calibration curves were generated from different standard concentrations of TMPP tags to establish the correlation between the integrated signal of fragments Frag-H6, 15, 24 and 33 and the concentration of the released TMPP tags in the reaction mixture, thus permitting to follow the release of TMPP tags from the mAbs.

Kinetic constants *k* were determined using the integrated rate equation for second order reaction. $ln\left(\frac{[A_0][B]}{[B_0][A]}\right) * \frac{1}{([B_0]-[A_0])} = k * t$ ; with $[A_0]$ and $[B_0]$ = initial concentrations of respectively TMPP-mAbs conjugates and DBCO-Acid, [A] and [B] = concentration at time t of respectively TMPP-mAbs conjugates and DBCO-Acid. [A] and [B] are calulated using the equations $[A] = [A_0] - x$ and $[B] = [B_0] - x$, with $x$ = measured concentration of released TMPP tag. The calculated kinetic constant *k* is the slope of the linear plot of $ln\left(\frac{[A_0][B]}{[B_0][A]}\right) * \frac{1}{([B_0]-[A_0])}$ versus time.

Kinetic constants were calculated by plotting ln(( 〖[A]〗 _0][B])/( 〖[B]〗 _0][A]))*1/(( 〖[B]〗 _0]- 〖[A]〗 _0]) ) versus time for the first 1000 seconds of the reaction. Each experiment was performed in triplicate.

**Table 2.** Kinetic rate constants values

| **mAb-TMPP** | **BVZ-H6** | **DUR-H15** | **CTX-H24** | **TRZ-H33** |
|---|---|---|---|---|
| **k ($M^{-1}.sec^{-1}$)** | 138.9 ± 14.3 | 324.7 ± 31.9 | 211.9 ± 34.4 | 85.1 ± 7.1 |

All analyses were performed on a Xevo G2-XS Q-Tof mass spectrometer, coupled with ultra-high-performance liquid chromatography (Acquity I-Class), both from Waters. The separation of released TMPP tags and antibodies was achieved on a BEH C18 column (50 × 2,1 mm ; 1,7 μm) at 40 °C. The mobile phase consisted of solvent A (water with 0.1% formic acid) and solvent B (acetonitrile with 0.1% formic acid) at a flow rate of 800 μL.min-1. The elution program was set in an isocratic mod with 65% solvent A for a duration of 90 seconds. The injection volume was set to 5 μL and the autosampler temperature at 37 °C.

The parameters of the electrospray ion source (ESI) were: Capillary voltage 3,5 kV ; sampling cone voltage 15 V ; source temperature 140 °C ; desolvatation gas (N2) temperature and flow rate 550 °C, 1000 UH ; cone gas flow rate 100 UH.

MS/MS data were acquired with the software Masslynx (Waters) in the positive ionization mode over a mass range of m/z 50 - 1200 Da. Collision energy was set to 55 V. Mass accuracy within 3 ppm was reached using a solution of leucine enkephalin for internal calibration. TargetLynx (Waters) software was used to construct calibration curves and for automatic calculation of the released TMPP tag concentrations. Integrations were automatically performed on fragments Frag-H6, 15, 24 and 33 m/z XIC with a mass window of 20 mDa.

In vitro experiments:

Materials

Bovin serum albumin (BSA), ammonium Chloride ($NH_4C$ l≥ 99.5%), phosphate buffered saline (PBS) tablette, bovin serrum albumin (BSA), tween 20, triton, paraformaldéhyde, acetone, Bicinchoninic Acid Protein Assay Kit, 4',6-diamidino-2-phénylindole (DAPI, $C_{16}H_{15}N_5$, 50.25 g.mol$^{-1}$), Dibenzocyclooctyne-PEG'-Fluor 545 (DBCO-Tamra, $C_{54}H_{57}N_50_{10}$, 936.09g/mol), Dibenzocyclooctyne-acid (DBCO-acid, $C_{21}H_{19}N0_3$, 333.38g/mol) were purchased from Sigma-Aldrich (France). Methanol and 2-Methylbutan were purchased from Carlo Erba (France) and Honeywell (France), respectively. CellBrite Fixe 640 Kit were purchased from Biotium (USA). SuperFrost Ultra Plus TM slides and 96-well plate were obtained from FisherScientific (France) and Greiner bio one (France), respectively. Normal (0.9%) saline and isoflurane were purchased from Baxter (France). Dulbecco's phosphate buffered saline (D-PBS, 10mM), HEPES buffer,

Trypsin-EDTA, Antibiotic-antimycotic, fetal bovine serum (FBS) and cell culture medium (DMEM, Dulbecco's Modified Eagle's medium or McCoy's 5A) were purchased from Gibco (Thermo Fisher Scientific, France). Matrigel matrix basement membrane was purchased from Discovery Labware. Erbitux (Cetuximab, CTX, 5 mg/mL), Bevacizumab (BVZ, 25 mg/mL), Durvalumab (DUR, 50 mg/mL) and Herceptin (trastuzumab, TRZ, 150 mg) were purchased from Merck (Belgium), Pfizer (USA), AstraZeneca (United Kingdom) and Roche (Switzerland). All products were used as received without further purification. Distilled water was purified using a Milli-Q system greater than 18MΩ cm resistance (Millipore, France) for all immunohistological buffers.

Detection and quantification limits of TMPP-tags by UPLC-MS/MS

50 μL of the cell media were evaporated to dryness under nitrogen using a Turbovap LV evaporator maintained at 40°C (Biotage, UK). Dried residue was then resuspended with 50 μL of a mixture of water/methanol (70/30) with 0.1% formic acid. The whole was vortexed 15 sec. and centrifuged 10 min at 20.000 g and supernatant was transferred into a vial for analysis.

10 μL of supernatant maintained at + 4°C was injected into the LC-MS/MS system which consist on a Waters ACQUITY I-Class UPLC® System with an Acquity BEH C18 2.1 × 50 mm, 1.7 μm, 130 Å pore size column and a reversed phase gradient over a run time of 5.5 min. A mix of mobile phase A (water with 0.1% formic acid) and mobile phase B (methanol with 0.1% formic acid) were used at a flow rate of 0.5 mL/min and a column temperature of 60°C. The gradient conditions ramped from 0% to 100% B between 0.5 and 3.0 min and then return to 0% B at 3.51 and were maintained up to 5.5 min for re-equilibration. In these conditions mean retention time was around 2.20 min for all TMPP-tags.

The mass spectrometry analysis was performed on a Waters XEVO™ TQ-S Mass Spectrometer operating in positive ion electrospray MRM mode. Three transitions were monitored for each TMPP-tags. Quantification was performed by averaging the 3 concentrations thus obtained. Multiple transitions monitored were *m/z* 750.95>180.0, 708.1, 734.15, 760.46>184.1, 717.26, 743.29, 769.46>189.13, 726.29, 752.22 and 778.12>191.87, 733.81, 759.46 for TMPP-H33, TMPP-H24, TMPP-H15 and TMPP-H6, respectively.

Quantification were performed using linear regression with 1/X^2 weighing. Lower limit of quantification (LLOQ) was determined as the lower concentration calculated by linear regression showing a maximum deviation of ± 20%. Limit of detection was determined as concentration which gave a signal-to-noise ratio of about 3.

**Table 3.** Limits of detection and of quantification of TMPP tags

| | Detection limit (pM) | Quantification limit (pM) |
|---|---|---|
| **TMPP-H33** | 20 | 65.3 |
| **TMPP-H24** | 25 | 73.6 |
| **TMPP-H15** | 20 | 66.3 |
| **TMPP-H6** | 25 | 74.5 |

Cell culture

Human epidermoid carcinoma (A431), lung adenocarcinoma (H1975) and glioblastoma (U87MG) were purchased from Sigma-Aldrich. Human breast adenocarcinoma (SkBr3) was furnished by the laboratory of Molecular and Cellular Therapeutic Engineering & Glycosyltransferases from the University of Lorraine (France). Cells were cultured in a humidified incubator (Sanyo, Japan) at 37 °C in an atmosphere containing 5% of CO2 in complete medium i.e. DMEM or McCoy's 5A supplemented with 10% of heat-inactivated FBS (Fetal Bovine Serum) and 1% antibiotic-antimycotic (Streptomycine, amphotéricine B, pénicilline). Mycoplasma absence was confirmed using MycoAlertTM kit (Lonza, USA).

Competitive binding assay:

Binding assay were conducted to determine the impact of the TMPP-tags functionalization on the antibody affinity to their cell membrane receptors. The binding assay was performed via two technics: by fluorescence analysis and by mass spectrometry analysis.

- By fluorescence analysis (figure 6)

Briefly, a low concentration of CTX-H24 (10 μg, 133.33 nM, 0.066 nmol) with or without a pharmacologic dose of CTX (100-fold excess of CTX) were added to 750 000 cells suspended in 500 μL of DMEM. After 2 hours incubation at 37

°C or 4 °C, the cells were centrifugated and washed three times with 1 mL of DMEM to remove unbound antibody. The cells were resuspended in 500 µL of DMEM and incubated with 10 equiv. of DBCO-TAMRA (1.4 µM in DMEM containing 1.4% DMSO) at 37 °C for 5 minutes under stirring. Then, cells were washed again 3 times with PBS. Finally, the fluorescence of the pellets was measured in a UV-visible spectrophotometer ($\lambda_{ex}$ = 450 nm). Control samples without CTX-H24 was also measured. Samples were done in triplicate.

- By mass spectrometry analysis (figure 7)

Briefly, low concentration of TRZ-H33, CTX-H24, DUR-H15, BVZ-H6, (1 µg, 13.33 nM, 0.0066 nmol), with or without a pharmacologic dose of CTX (100-fold excess of CTX) were added to 250 000 cells suspended in 500 µL of DMEM (For TRZ: SkBr3, for CTX: A431, for DUR: H1975, for BVZ: U87MG). After 2 hours incubation at room temperature with continuous stirring, the cells were centrifugated and washed three times with 1 mL of DMEM to remove unbound antibody. The cells were re-suspended in 500 µL of DMEM and incubated with large excess of DBCO-acid (75 757 equiv., 1 mM in DMEM containing 1% DMSO) at 37°C for 20 minutes under stirring. Then, cells were harvested by centrifugation and the supernatants were stored at -20°C until mass spectrometry analysis. Samples were done in triplicate.

**Table 4** summarizing the concentrations of released Tags.

| | **TRZ-H33** | **TRZ-H33 + 100xTRZ** | **CTX-H24** | **CTX-H24 + 100xCTX** | **DUR -H15** | **DUR-H15+ 100xDUR** | **BVZ-H6** | **BVZ-H6 + 100xBVZ** |
|---|---|---|---|---|---|---|---|---|
| **MS-Tag Name** | **TMPP-H33** | **TMPP-H33** | **TMPP-H24** | **TMPP-H24** | **TMPP-H15** | **TMPP-H15** | **TMPP-H6** | **TMPP-H6** |
| **[MS-Tag] released** | 2753±248 pM | 1859±347 pM | 103±20 pM | 43±15 pM | 206±43pM | 56±7pM | 392±145pM | 155±24pM |

Fluorescent microscopy experiments: targeting CTX-H24 with DBCO-TAMRA in live cells.

A431 cells were seeded in 8-well Nunc Lab-Tek I chambers (150000 cells per well in 500 µL of DMEM) one day before the experiment. The medium was then changed, and cells were incubated with CTX-H24 (5 µg, 66.67 nM, 0.033 nmol) in 500 µL of DMEM for 2 h at 37°C. The cells were washed three times with DMEM, re-suspended in 500 µL of DMEM and incubated with 100 equivalents of DBCO-TAMRA (6 µM in 500 µL of DMEM containing 0.6% DMSO) at 37 °C for 15 min. Cells were washed again three times with DMEM and cells were incubated with DAPI (10 µg/mL or 30 µM in DMEM) to stain the nucleus during 15 min at 37 °C. Cells were washed one time with PBS. CellBrite Fix 640 was used to stain the membrane 1X PBS 15 min at 37°C. Cells were washed three times with PBS. Cells were fixed in neutral buffer formalin 10 % for 20 min at RT. Cells were washed three times with PBS before imaging. Control samples without CTX-H24 or without CellBrite Fix 640 were also imaged.

Fluorescence microscopy was performed on an AxioObserver Z1 microscope (Zeiss) using two objectives (X20 o X40) with relevant filters :

- Long pass filter ($\lambda_{exc.}$ = 405 nm and $\lambda_{em.}$ = 454 nm) for DAPI
- Band Pass filter ($\lambda_{exc.}$ = 553 nm and $\lambda_{em.}$ = 575 nm) for TAMRA
- Band Pass filter ($\lambda_{exc.}$ = 638 nm and $\lambda_{em.}$ = 667 nm) for CellBrite Fix 640.

Experiment on tissues :

Serial tumor sections (14 µm thick) were cut at -20°C with a cryostat (Leica) on SuperFrost Ultra Plus TM slides. Frozen tumor slides were fixed in neutral buffer formalin 10 % for 15 min at RT, then washed 5 min with PBS buffer. The neutral buffer formalin 10 % effect were inactivated by incubating the sections in NH4Cl solution (50 mM in PBS), then washed 5 min with PBS buffer. Sections were successively immersed during 5 min at RT in permeabilized solutions: MeOH/acetone (50/50) and Triton solution (0.1% in PBS), then washed 5 min with PBS buffer.

Sections were immersed in blocking solution (5% BSA in Tween-20 solution of 0.5 % in DMEM without phenol red) for 1h at RT. Sections were incubated 2 h at RT with a low concentration of CTX-H24 alone (1 µg, 13.33 nM, 0.0066 nmol) or in the co-presence of 100-fold excess of CTX in Tween-20 solution of 0.5 % in DMEM without phenol red. After washing three times with DMEM without phenol red, sections were incubated 20 min at 37°C with DBCO-acid (75 757, 10 mM in DMEM containing 10% DMSO). The supernatant was harvested and stored at -20°C until mass spectrometry analysis.

Ex vivo: proteins of interest expression on the A431 implanted cells by immunofluorescence.

Snap-Frozen A431 tumor were cut in 10μm slices. The slices have been fixed in 10% formalin (Sigma-Aldrich # HT501128-4L) during 15 minutes at room temperature (RT) for immunofluorescence for protein analysis. All the slices were incubated during 5 minutes in PBS Triton buffer to permeabilized the tissue. After bovine serum albumin blocking (BSA, ThermoFisher), the slices were washed with cold PBS and then incubated with the different antibodies of interest:

- EGFR : 43B2 monoclonal Rabbit from Abcam (dilution: 1/100)
- ErbB2 : MA514509 monoclonal Rabbit from Fisher Scientific (dilution: 1/250)
- PD-L1 : E1L3N monoclonal Rabbit from CellSignaling (dilution: 1/100)
- VEGF : ABS82 Polyconal Rabbit from Sigma Aldrich (dilution: 1/200)

Slices were washed three times with PBS buffer between each step. The protein expression was determined with a secondary antibody (2-Goat anti-Rabbit, Alexa Fluor™ 546, dilution: 1/1000). Sections were mounted using Prolong Diamond Antifade Moutain with DAPI to label cell's nucleus. An Axio Observer Z1 microscope (Zeiss, Germany) was used with a 20x objective to scan the full sections. Post processing qualitative analysis was performed with ZEN software (v2.6, Zeiss).

Statistical analysis

All data are presented as mean ± standard deviation (SD). The statistical analyses were performed using GraphPad Prism software (Graph Pad software Inc., San Diego, USA). Normality was assessed using the d'Agostino-Pearson test. Unpaired t-test were used for statistical significance of intergroup comparisons (*$p<0.05$).

In vivo experiments

Ethics and animals

Animal experiments were conducted in agreement with the European Directive 2010/63/EU on the protection of laboratory animals (French law transposition: Decree No. 2013-118). They were performed at the imaging facility from BioMaps and DMTS with protocols approved by the Ethical Committee of CEA (CEtEA, authorization CEEA N° 44).

Subcutaneous tumor model

Mice anesthetized with isoflurane in a mixture of $N_2/O_2$ [80:20] (4% for induction and 2% for maintenance) received a subcutaneous injection of A431 cells at $5.0 \times 10^6$ tumor cells suspended in 100 μL of PBS: Matrigel (50%/50% v/v) in both right and left flanks for heterotopic establishment of tumors. Animal weight and tumors growth was monitoring three times a week. When the tumor reached 1 cm in diameter after approximately 20 days, mice were euthanized by cervical dislocation under isoflurane (5%). Tumors were removed, immersed in 2-Methylbutan and frozen in liquid nitrogen.

Biodistribution of [$^{18}$F]-DBCO after intratumoral injection (figure 8)

In total, 8-female athymic NMRI nude mice five-week-old were purchased from Janvier laboratories (Le Genet sur Isle, France, Mus musculus, NMRI-FOXN1 Nu/Nu). Mice were housed four per cage with food and water *ad libitum* in an environmental enrichment (polycarbonate cottages and wooden stocks), in a temperature (22 ± 2 °C) and humidity (40%) controlled room and were maintained under specific pathogen-free conditions.

[$^{18}$F]-DBCO was prepared according to our previously reported protocol [18]. Briefly, the radiosynthesis was carried out using TRACERlab FXFN or FXNPro synthesizer starting with [$^{18}$F]fluoride trapping on an anion exchange cartridge, then eluted with a solution of $K_2CO_3$ (2 mg) and K222 (12-15 mg) in a water/MeCN 30:70 solution (1 mL). After evaporation, the corresponding tosylate precursor (5 mg) in MeCN (1 mL) was added and the mixture heated to 90 °C for 15 minutes. The crude mixture was purified by semi-preparative HPLC (Zorbax SB C18 column, water/MeCN 35:65, 0.1% TFA) and the eluate was reformulated in ethanol (1.5 mL) with a C18 cartridge. The final molar activity was 193 GBq.μmol$^{-1}$. Before injection, the DBCO concentration was reajusted to 100 mM. The dose injected by mouse was 9.5±2.3 MBq, 10 μL.

In vivo pharmacokinetics of TMPP tags

6 NMRI nude mice (female, 5 weeks) were implanted with the A431 in each flank (right and left), with 5 $5.0 \times 10^6$ cells per injection. The weight of the mice and the size of the tumors were measured regularly over time. 21 days post

implantation after the tumor growth, the mice were injected intravenously with the cocktail of four distinct tags at 0.2 mg/kg for each tag (H33, H24, H15, H6, total volume: 120 μL). Then, blood samples were collected (retro-orbital) at 30 min, 1 h, 2 h, 4 h, and 24 h after injection, on three distinct mice at each time. The plasma was separated thanks to a centrifugation of the blood in heparin tube at 20°C, 15000 g, 15 min, and conserved at -20°C before LC-MS analysis. Urine samples were collected after 24 h in metabolic cage on three mice, and conserved at -20°C before LC-MS analysis. Quantification of tags contained in plasma and urine was performed using UPLC-ESI (+)-MS/MS method. Animals were euthanized 24 h after tags administration.

In vivo immunoprofiling :

Two experiments have been performed to assess Tags release in plasma, blood and tumors at various time after injection.

For the first experiments, 6 NMRI nude mice (female, 5 weeks) were implanted with the A431 in each flank (right and left), with $5.0 \times 10^6$ cells per injection. The weight of the mice and the size of the tumors were measured regularly over time. 21 days post implantation after the tumour growth, the mice were injected intravenously with the cocktail of four antibodies, TRZ-$H_{33}$, CTX-$H_{24}$, DUR-$H_{15}$, BVZ-$_{H6}$ (100 μg per antibody, total volume: 120 μL). 3 days post-injection, the tumors were injected with 10 μL of a DBCO solution (100 mM in DMSO/$H_2O$-NaCl 0.9% 30/70). Blood sampling via retro-orbital protocol was performed under anesthesia (2% of isofluorane, in $O_2$) at 30 min, 1 h, 2 h and 24 h post-injection, on three distinct mice at each time. The plasma was separated thanks to a centrifugation of the blood in heparin tube at 20°C, 15000 g, 15 min. The urine of three mice was collected during 24 h thanks to individual metabolic cages. The plasma and urine were stored at -20°C before LC-MS processing. At 24 h post DBCO injection, all the mice were euthanized for ex vivo MS analysis on various shredded organs of interest (blood, kidneys, liver and tumors).

For the second experiments, 4 NMRI nude mice (female, 5 weeks) were implanted with the A431 in each flank (right and left), with $5.0 \times 10^6$ cells per injection. The weight of the mice and the size of the tumors were measured regularly over time. 21 days post implantation after the tumour growth, the mice were injected intravenously with the cocktail of four antibodies, TRZ-$H_{33}$, CTX-$H_{24}$, DUR-$H_{15}$, BVZ-$H_6$ (100 μg per antibody, total volume: 120 μL). The urine was collected thanks to metabolism cages during the 3 days beofre DBCO injection. 3 days after mAb cocktail injection, the left tumor of each mice was injected with 10 μL of a DBCO solution (100 mM in DMSO/H2O-NaCl 0.9% 30/70). The urine of three mice was collected during 24 h thanks to individual metabolic cages. Blood sampling via retro-orbital protocole was performed under anesthesia (2% of isofluorane, in $O_2$) before the mAb cocktail injection, 48 h after mAb injection and after DBCO injection at 30 min and 24 h post-injection, on the four distinct mice at each time. The plasma was separated thanks to a centrifugation of the blood in heparin tube at 20°C, 15000 g, 15 min. The plasma and urine were stored at -20°C before LC-MS processing. At 24 h post DBCO injection, all the mice were euthanized for ex vivo MS analysis on various shredded organs of interest (blood, kidneys, liver and tumors).

LC-MS/MS analysis of samples from *in vivo* experiments: Tags H33, H24, H15 and H6 quantification in growth medium and in mouse plasma, kidney, liver, urine and tumor.

All solvents (VWR France) were LC/MS grade. 50 μL of growth medium was evaporated to dryness using a Turbovap LV evaporator maintained at 40°C (Biotage, UK). Extract was then resuspended with 50 μL of water/acetonitrile (70/30, V/V) with 0.1% formic acid. At last, debris were removed by spinning 5 min at 20,000 g and 5°C and supernatant was transferred into a new vial for analysis.

Kidney, liver and tumor samples were homogenized with three volumes of water using a Bertin Technologies Precellys homogenizer before analysis. Then, 200 μL of acetonitrile was added in 50 μL of tissue homogenate (kidney, liver or tumor), plasma for the protein precipitation or urine. The whole was vortexed 15 s and centrifuged 15 min at 20,000 g and 5°C. Extract was then resuspended with 50 μL of water/acetonitrile (70/30, V/V) with 0.1% formic acid. At last, debris were removed by spinning 10 min at 20,000 g and 5°C and supernatant was transferred into a new vial for analysis.

Ten μL of extract maintained at +5°C was injected into the LC-MS/MS system which consist on a Waters ACQUITY UPLC® System with an Acquity UPLC BEH C18 2.1*50 mm column and a reversed phase gradient over a run time of 5.5 minutes. A mix of mobile phase A (water and 0.1% formic acid) and mobile phase B (methanol and 0.1% formic acid) were used at a flow rate of 0.5 mL/min and a column temperature of 60°C. The gradient conditions ramped from 0% B to 100% B between 0.5 and 3 min and maintained up between 3 and 3.5 min, ramped to 5% B at 3.51 min and maintained up to 5.5 min for re-equilibration. The mass spectrometry analysis was performed on a Waters XEVO™ TQ-S Mass Spectrometer operating in positive ion electrospray MRM mode. Multiple transitions monitored were $m/z$ 751.0 > 180.01, 760.5 > 184.1, 769.5 >189.1 and 778.1 > 733.8 for TAG H33, H24, H15 and H6, respectively. In these conditions mean retention time was around 2.2 min for each Tags.

Quantification was performed using linear regression with $1/X^2$ weighing and calibration ranges from 0.5 to 100 ng/mL, for each Tags.

Results and discussion

Preparation of MS-Tagged antibodies

The synthetic approach followed for the synthesis of MS-tags allows easy access to at least 15 among the 71 possible labelled TMPP (figure 3). As a proof of concept, we performed our study with 4 isotopologues of TMPP which were synthesized in 6 steps from iminosydnone 1, prepared according to previous protocols [19]. These four isotopically labelled TMPP tags were attached to antibodies through iminosydnone cleavable linkers described in [20]. Iminosydnonimines are mesoionic compounds described by our group to undergo bioorthogonal Strained-Promoted SydnonImine-Cyclooctyne cycloaddition (SPSIC) reactions ([21]). Upon SPSIC reaction, iminosydnones are rapidly and quantitatively cleaved to release an urea-TMPP product and to form a pyrazole product clicked to the antibody (Figure 2). Short PEG spacers were introduced into the sydnonimine core to form compound **3** whose alcohol function was then tosylated to allow reaction with trimethoxybenzene phosphine and three of its deuterated analogues to obtain four phosphoniums **5** bearing TMPP isotopologues, TMPP-H33, H24, H15 and H6 (Figure 4).

Using standard peptide coupling (figure 5), the TMPP tags were then attached to four FDA-approved therapeutic antibodies (mAbs-TMPP) raised against biomarkers located at the surface of the cell: Trastuzumab (TRZ, anti-Her2), Cetuximab (CTX, anti-EGFR), Durvalumab (DUR, anti-PDL1) and Bevacizumab (BVZ, anti-VEGF). TRZ, CTX, and DUR target extracellular tumour receptors, whereas BVZ targets the growth factor VEGF-A secreted by the cells and bound at their surface. The significant presence of VEGF-A at the surface of tumoral cells has been evidenced by numerous studies [22]. The number of TMPP linked to the antibodies, TAR for Tag Antibody Ratio, was determined (TAR from 2 to 4, Figure 4 and Table 1). To control the reactivity of the iminosydnone linker, the cleavage of each tagged antibodies was performed in solution using the SPSIC reaction. The monitoring of the reaction was carried out by LC-HRMS measurements of the released tags. A quantitative detachment of the TMPP tags from the antibodies was observed in a few minutes using excess of the cyclooctyne DBCO. High kinetic constants values k were found in all cases: between 85 to 325 $M^{-1}.s^{-1}$ depending on the antibody (Figure 4).

The limits of detection and quantification in solution of each TMPP-tag were then determined by LC-MS/MS analysis and found to be ~20 pM and ~70 pM respectively in DMEM cell media (Table 3).

Demonstration of the method’s feasibility

Having these tools ready, a series of experiments were first carried out for assessing the correlation between the quantification of released TMPP tags and antigen expression. To this end, *in vitro* experiments were performed using CTX-H24 as model TMPP-mAb and the human epidermoid carcinoma cells A431 that overexpress the epidermal growth factor receptor (EGFR), as confirmed by western blot analysis (not shown). CTX-H24 was incubated 2 h with A431 cells and then DBCO-TAMRA was added and keep to react during 15 min. Epifluorescence microscopy confirmed a specific fluorescent signal at the cell membrane highlighting the efficient click reaction of DBCO-TAMRA to CTX-H24 bound to A431 cells (not shown). However, a slight non-specific signal coming from the DBCO-TAMRA probe was also observed into the cell cytoplasm. Co-localization experiments conducted with a membrane tracker (Cell Brite Fix 640) confirmed the effective TAMRA-labelling of Cetuximab bound to the EGFR embedded into the cell membrane. The specific versus non-specific binding were evaluated by fluorescence measurements of CTX-H24 displacement by a high dose of unlabeled CTX (Figure 8B).

The results showed high and displaceable binding of CTX-$H_{24}$ to A431 either at 37°C or 4°C, and thus confirmed that CTX keeps its ability to specifically interact with EGFR present on cell membrane after the introduction of the TMPP-$H_{24}$ tag. According to microscopy experiments, when cells were incubated with DBCO-TAMRA alone, a significant background signal was observed due to non-specific binding of DBCO to the cell. However, this phenomenon does not alter the selective release of the MS-tag TMPP-$H_{24}$ from CTX. A noticeable increase of fluorescence was clearly observed when DBCO-TAMRA was added after incubation of CTX-$H_{24}$ and $H_{24}$ was easily detected and quantified by MS (Figure 8C). The binding of CTX-$H_{24}$ to the EGFR present on A431 was significantly displaceable by excess of CTX (p = 0.0024) which confirmed the specificity of the reaction: quantification of TMPP-$H_{24}$ tag was ~43 pM and -103 pM with and without excess of CTX respectively. A saturation binding assay using released TMPP-$H_{24}$ detection was performed to determine the apparent dissociation constant ($K_d$) and the maximum number of binding sites (Bmax). Around 6.6.106 EGFR receptors per cell in the A431 cell line were quantified (Figure 7A), a value in coherence with literature data [23]. The conjugation of TMPP-$H_{24}$ to CTX has been shown to have minimal impact on CTX binding when compared to its fluorescent counterpart (Figure 7B and C). It was then verified the preservation of the binding properties and quantified the released TMPP tags of the three other TMPP-mAbs, namely TRZ-$H_{33}$, DUR-$H_{15}$ and BVZ-$H_6$. LC-MS/MS quantification of each TMPP-tags after addition of DBCO on TMPP-mAbs incubated with cells overexpressing the specific receptor: SkBR3, H1975 and U87 cell lines respectively, confirmed the good binding properties of each bioconjugates and the specific detection of each receptors (Figure 7A).

Immuno-profiling on tissue sections

Following this validation study, a series of experiments were carried out with the goal to quantify several receptors simultaneously on biopsy tissues. This multiplex approach consists at adding the mixture of the four labelled antibodies on tissue slices (14 μm thick) from mice bearing subcutaneous A431 tumours and to quantify the four released TMPP tags after addition of DBCO. The cocktail of the four TMPP-mAbs, TRZ-$H_{33}$, CTX-$H_{24}$, DUR-$H_{15}$ and BVZ-$H_6$ was thus incubated on tissue sections and the abundance of each released TMPP-tags fast determined by MS after addition of DBCO (Figure 9). Three adjacent tumour sections from an A431 subcutaneous tissue were pulled together in aim to be quantified by LC-MS/MS analysis. Fluorescence imaging after addition of DBCO-TAMRA confirmed efficient and selective click-and-release reaction on tissue sections (Figure 9B). As shown in Figure 9C, LC-MS/MS analysis confirmed that TMPP-$H_{24}$ is the most abundant representing more than 85% of all released tags. This result confirmed that EGFR was present at higher degree on cell membrane compared to the other three receptors. Control experiments conducted with excess of CTX confirmed the specificity of EGFR quantification and immunofluorescence imaging of the tissue sections confirmed the overexpression of CTX compared to the other receptors (not shown).

These results showed that the present approach can be used to profile tumor biopsies by detecting several biomarkers in one single process.

Immuno-profiling in vivo: proof of concept

Finally, it was decided to evaluate if the present click-and-release strategy might work *in vivo.* This is a tremendous challenge as it involves the intravenous (i.v.) injection of the antibody cocktail followed, 3 days later, by the injection of the cyclooctyne. For the strategy to be successful, the cyclooctyne should react *in vivo* with tagged antibodies fixed at the tumor site, and not with circulating antibodies, detach the tags which then must be, at least partially, excreted in urines in order to be detected and quantified by LC-MS/MS. The obstacles of this approach are therefore extremely difficult to overcome but the stakes are very high. Indeed, biopsy analysis may not fully reflect the state of an entire tumor which may be heterogeneous. To date, there is no method allowing multiplexed in vivo analysis of biomarkers expression and it was aimed to see if the present method could possibly meet this need. To limit the obstacles to overcome, it was decided to use intratumoral injection of the cyclooctyne (Figure 10A). This type of injection allowed concentrating the cyclooctyne at the tumor site and limits its diffusion as showed by PET imaging of mice after intratumoral injection of [$^{18}$F]-labelled DBCO 6 (Figures 10B). Most of the DBCO uptake remained on the tumor even 4 hours post-injection. Pharmacokinetics of TMPP-tags were also carried out by blood samplings and showed fast blood clearance of the tags and sufficient excretion in urine to be detected and quantified by LC-MS/MS after 24 h.

Importantly, the four tags displayed same pharmacokinetics and excretion profiles (Figure 11). Reinforced by this observation, mice bearing left and right A431 tumors were injected intravenously with a cocktail of the four TMPP-mAbs (TRZ-$H_{33}$, CTX-$H_{24}$, DUR-$H_{15}$ and BVZ-$H_6$, 100 μg each). Three days post-injection, which correspond to the maximum of antibodies accumulation into the tumor, an excess of non-radiolabeled DBCO [$^{19}$F]-6 was administrated via intratumoral injection in the left tumor. 24 hours later, the urine sampling revealed an important release of the TMPP-$H_{24}$ compared to the TMPP-$H_{33}$ and TMPP-$H_6$ tags that can be correlated to the higher expression of EGFR in A431 model To confirm these *in vivo* results, the tumors were collected and the concentration of TMPP-tags that were still inside the tumor tissue was measured. It was found similar profile as the one obtained in urine excretion (Figure 10C).

The concentration of the tags in the right tumor (which did not receive DBCO injection) was markedly lower, reaching the limit of MS detection. This observation highlights a limited diffusion of the DBCO following intratumoral injection (not shown). Urine does not contain any traces of tags before [$^{19}$F]-6 injection, indicating good in vivo stability of the antibody-tag conjugates (not shown).

The urine concentration of the Tag, obtained through metabolism cages, may thus offer a more representative measure of Tag release compared to blood samples. This is because the rapid elimination kinetics of the tag from the blood compartment make it challenging to accurately assess the total tag release (not shown).

**BIBLIOGRAPHIC REFERENCES**

[1] D. Zahavi, L. Weiner, Antibodies 2020, 9, 34.

[2] A. Beck, L. Goetsch, C. Dumontet, N. Corvaïa, Nature Reviews Drug Discovery 2017, 16, 315;

[3] Y. Imai, C. K. Leung, H. G. Friesen, R. P. Shiu, Cancer Res. 1982, 42, 4394;

[4] R. Weissleder, M. C. Schwaiger, S. S. Gambhir, H. Hricak, Sci. Transl. Med. 2016, 8, 355ps16

[5] S. C. Bendall, E. F. Simonds, P. Qiu, El-ad D. Amir, P. O. Krutzik, R. Finck, R. V. Bruggner, R. Melamed, A. Trejo, O. I. Ornatsky, R. S. Balderas, S. K. Plevritis, K. Sachs, D. Pe'er, S. D. Tanner, G. P. Nolan, Science 2011, 332, 687

[6] W. Schubert, B. Bonnekoh, A. J. Pommer, L. Philipsen, R. Bçckelmann, Y. Malykh, H. Gollnick, M. Friedenberger, M. Bode, A. W. M. Dress, Nat. Biotechnol. 2006, 24, 1270.

[7] M. J. Gerdes, C. J. Sevinsky, A. Sood, S. Adak, M. O. Bello, A. Bordwell, A. Can, A. Corwin, S. Dinn, R. J. Filkins, D. Hollman, V. Kamath, S. Kaanumalle, K. Kenny, M. Larsen, M. Lazare, Q. Li, C. Lowes, C. C. McCulloch, E. McDonough, M. C. Montalto, Z. Pang, J. Rittscher, A. Santamaria-Pang, B. D. Sarachan, M. L. Seel, A. Seppo, K. Shaikh, Y. Sui, J. Zhang, F. Ginty, Proc. Natl. Acad. Sci. USA 2013, 110, 11982; b) J. R. Lin, M. Fallahi-Sichani, P. K. Sorger, Nat. Commun. 2015, 6, 8390.

[8] R. M. Schweller, J. Zimak, D. Y. Duose, A. A. Qutub, W. N. Hittelman, M. R. Diehl, Angew. Chem. Int. Ed. 2012, 51, 9292;

[9] M. Mondal, R. Liao, L. Xiao, T. Eno, J. Guo. Angew. Chem. Int. Ed. 2017, 56, 2636.

[10] J. Ko, J. Oh, M. S. Ahmed, J. C. T. Carlson, R. Weissleder, Angew. Chem. Int. Ed. 2020, 59, 6839.

[11] M. Ribéraud, E. Porret A., Pruvost, F. Theodoro, A. L. Nguyen, S. Specklin, D. Kereselidze, C. Denis, B. Jego, P. Barbe, M. Keck, T. D'anfray, B. Kuhnast, D. Audisio, C. Truillet and F. Taran, J. Am. Chem. Soc. 2023, 145, 4, 2219-2229.

[12] Tang KC et Raj M. Angew. Chem. Int. Ed. 2021, 60, 1797- 1805.

[13] Paramelle D. et al, Angew. Chem. Int. Ed. 2010, 49, 8240-8243.

[14] Li S. etal, Anal. Chem. 2019, 91, 1701-1705**.**

[15] Stevens KG et Pukala T.L, Trends in Analytical Chemistry 132 (2020) 116064.

[16] Goydel RS et Rader C. Oncogene 2021 May;40(21):3655-3664.

[17] Passaro A.et al, Cell 187, 2024.

[18] Richard, M.; Truillet, C.; Tran, V. L.; Liu, H.; Porte, K.; Audisio, D.; Roche, M.; Jego, B.; Cholet, S.; Fenaille, F.; Kuhnast, B.; Taran, F.; Specklin, S. Chem. Commun. 2019, 55, 10400-10403.

[19] M. Riomet, K. Porte, L. Madegard, P. Thuéry, D. Audisio, F. Taran, Org. Lett. 2020, 22, 6, 2403.

[20] M. Riomet, E. Decuypere, K. Porte, S. Bernard, L. Plougastel, S. Kolodych, D. Audisio, F. Taran, Chem. Eur. J. 2018, 34, 8535.

[21] S. Bernard, D. Audisio, M. Riomet, S. Bregant, A. Sallustrau, L. Plougastel, E. Decuypere, S. Gabillet, R. A. Kumar, J. Elyian, M. N. Trinh, O. Koniev, A. Wagner, S. Kolodych, F. Taran, Angew. Chem. Int. Ed. 2017, 56, 15612.

[22] W. B. Nagengast, M. N. Lub-de Hooge, S. F. Oosting, W. F. A. den Dunnen, F-J. Warnders, A. H. Brouwers, J. R. de Jong, P. M. Price, H. Hollema, G. A. P. Hospers, P. H. Elsinga, J. W. Hesselink, J. A. Gietema, E. G. E. de Vries. Cancer Research 2011, 71, 143.

[23] D. Patel, A. Lahiji, S. Patel, M. Franklin, X. Jimenez, D. J. Hicklin, X. Kang. Anticancer Res. 2007, 27(5A), 3355.

[24] Protopopov, Obshch. Khim.1963, 33, 3050-3052

## Claims

1. A probe specifically recognizing a target biological molecule of interest, said probe containing a probe part being linked to a detectable mass spectrometry enhancer (MS-tag) through a cleavable linker, said cleavable linker being an iminosydnone of formula I:

(I) 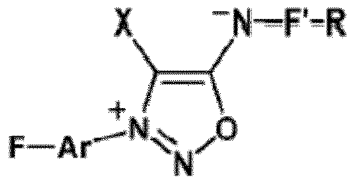

Wherein F is a functional group selected from the group consisting of: a carboxylic acid COOH group, a thiol SH group, a maleimide group, an activated ester, a halogen atom, an alkene or alkyne group, an amino (-NRR') group wherein R and R' are independently hydrogen atoms, alkyl, alkene, alkyne or aryl groups, a hydroxylamine ($-ONH_2$) group, a hydrazine ($-NH-NH_2$) group, an azido ($-N_3$) group, a diazonium ($-N_2^+$) group, a boronic acid $-B(OR")_2$ group wherein R" is a hydrogen atom or an alkyl group, an isocyanate (-N=C=0) or isothiocyanate (-N=C=S) group, a chlorosulfonyl ($-SO_2Cl$) group, a -C≡C-C≡N group, an aldehyde CHO group, a ketone COR‴ group wherein R‴ is an alkyl group, and an alkyl group substituted by at least one of said groups,
Wherein X is selected from the group consisting of a hydrogen atom, a halogen atom, an aryl diazo group, an alkyl group, an aryl group, an alkenyl group, an alkynyl group, an alkoxy group, a thioether group and an amino group,
Wherein Ar is an alkyl or NR2 group with R is an alkyl, an aryl, an acyl or a sulfonyl group, or an optionally substituted aromatic group,
Wherein F' is a carbonyl group (C=0), a sulfonyl group, an alkyl, an aromatic ring or a phosphoryl group (P=0), and
Wherein R is selected from an optionally substituted aryl group, an optionally substituted alkyl, alkenyl or alkynyl group, an optionally substituted alkoxy or aralkyloxy group, an optionally substituted thioether group, an optionally substituted amino group, wherein the alkyl, alkenyl and/or alkynyl groups may be interrupted by at least one heteroatom selected from nitrogen, oxygen and sulphur atoms and wherein said substituents are one or more groups selected from a carboxylic acid COOH group, a thiol SH group, a maleimide group, an activated ester, a halogen atom, an alkene or alkyne group, optionally interrupted by at least one heteroatom selected among O, N and S, an amino (-NRR') group, wherein R and R' are independently hydrogen atoms, alkyl, alkene, alkyne or aryl groups, a hydroxylamine ($-ONH_2$) group, a hydrazine ($-NH-NH_2$) group, an azido ($-N_3$) group, a diazonium ($-N_2^+$ ) group, a boronic acid $-B(OR")_2$ group, wherein R" is a hydrogen atom or an alkyl group, an isocyanate (-N=C=0) or isothiocyanate (-N=C=S) group, a chlorosulfonyl ($-SO_2Cl$) group, a -C≡C-C≡N group, an aldehyde CHO group, a ketone COR‴ group, wherein R‴ is an alkyl group.

Wherein said linker is configured to be cleaved in the presence of an alkynyl cleaving compound.

2. The probe of claim 1, wherein said probe part is chosen in the group consisting of: an antibody, an aptamer, an oligonucleotide, or any other probe specifically recognizing at least one protein, at least one oligonucleotide such as DNA or RNA, at least one small organic molecule or at least one another cellular component.

3. The probe of claim 1 or 2, wherein said iminosydnone of formula I is chosen in the group consisting of:

- (3-(4-carboxyphenyl)-1,2,3-oxadiazol-3-ium-5-yl)(methylcarbamoyl)amide ;
- (2-(4-hydroxyphenyl)- 1 ,2,3-oxadiazol-2-ium-5-yl)(p-tolylcarbamoyl)amide ;
- (2-(4-carboxyphenyl)-1,2,3-oxadiazol-2-ium-5-yl)(p-tolylcarbamoyl)amide ; and
- ((4-(ethoxycarbonyl)phenyl)carbamoyl)(2-(4-hydroxyphenyl)-1,2,3-oxadiazol-2-ium- 5-yl)amide.

4. The probe according to any of claims 1, 2 or 3, wherein said detectable MS-tag is tris(2,4,6,-trimethoxyphenyl)-phosphonium (TMPP) or an isotope thereof.

5. The probe according to any of claims 1-4, wherein said alkynyl cleaving compound is a cyclooctyne chosen in the group consisting of: bicyclo- [6.1.0]-nonyne (BCN), 3,3-difluorocyclooct-1-yne (DIFO), dibenzocyclooctyne (DBO) or said alkynyl cleaving compound is a cycloheptyne such as tetramethylthiacycloheptyne (TMTH), preferably said

alkynyl cleaving compound is dibenzoazacyclooctyne (DBCO).

**6.** The probe according to any of claims 1 to 5, wherein said probe part is an antibody specifically recognizing a protein cancer biomarker, said protein cancer biomarker being for example Her2, EGFR, PDL1 or VEGF.

**7.** A kit containing at least two sets of probes as defined in any of claims 1-6, wherein the probe parts within each set of probes specifically recognize the same target biomarker molecule, and are linked to the same MS-tag whose mass-to-charge ratio is specifically associated to said target biomarker molecule, and optionally a recipient containing an alkynyl cleaving compound such as dibenzoazacyclooctyne (DBCO).

**8.** *In vitro* use of at least one probe as defined in any of the claims 1-6 or of the kit as defined in claim 7, to detect the presence of at least one biomarker molecule that is characteristic of a disease or disorder, in a biological sample from a living animal, or to monitor the progression of a disease and/or the effect of a therapy, wherein said disease is preferably cancer.

**9.** The probe as defined in any of the claims 1-6 or the kit as defined in claim 7, for use to detect at least one biomarker molecule that is characteristic of a disease or disorder in living animals, or to monitor the progression of a disease and/or the effect of a therapy, wherein said disease is preferably cancer.

**10.** An *in vitro* method for detecting a biological molecule in a biological sample, comprising :

a) Incubating said sample including a target molecule with at least one probe as defined in claim 1-6,
b) Optionally washing the sample so as to remove the unbound probes,
c) Contacting the biological sample with an alkynyl cleaving compound that is able to cleave the linker contained in the bound probes, the cleaving compound being preferably as defined in claim 5,
d) Measuring the nature and/or the amount of the released MS-tags in the biological sample, by mass spectrometry,

wherein the nature and/or the amount measured in step d) reveals the presence and / or the amount of the biomarker molecule in the biological sample.

**11.** The *in vitro* method of claim 10, wherein it is a multiplex method for diagnosing a cancer in a patient in need thereof, said multiplex method preferably comprising the steps of:

a) Contacting said biological sample with at least two sets of probes as defined in claims 1-6,
b) Optionally washing the sample in order to remove the unbound antibodies,
c) Contacting the biological sample with an alkynyl cleaving compound that is able to cleave the linkers contained in the bound probes, the cleaving compound being preferably as defined in claim 5,
d) Measuring the nature and/or the amount of the released MS-tags in the biological sample, by mass spectrometry,

wherein the nature and/or the amount measured in step d) reveals the presence and / or the amount of the biomarker molecules in the biological sample and therefore permits cancer diagnosis of the patient from which the biological sample originates.

**12.** A method for detecting a biological molecule in a living animal in need thereof, said method comprising the steps of:

a) Administering to said living animal an effective amount of at least one probe as defined in any of the claims 1-6,
b) Administering to the living animal an alkynyl cleaving compound that is able to cleave the linker contained in the bound probes, the cleaving compound being preferably as defined in claim 5,
c) *In vitro* detecting, by mass spectrometry, the nature and/or the amount of the released MS-tags in a biological sample collected from the patient after step b),

wherein the nature and/or the amount measured in step c) reveals the presence and / or the amount of the biomarker molecule in the living animal.

**13.** The method of claim 12, wherein it is a multiplex method for diagnosing a cancer in a patient in need thereof, said multiplex method comprising the steps of :

a) Administering to said patient an effective amount of at least two sets of probes as defined in claims 1-6,
b) Administering to the patient an alkynyl cleaving compound that is able to cleave the linker contained in the bound probes, the cleaving compound being preferably as defined in claim 5,
c) *In vitro* detecting, by mass spectrometry, the nature and/or the amount of the released MS-tags in a biological sample collected from the patient after step b),

wherein the nature and/or the amount of levels of MS-tags measured in step c) reveals the presence and / or the amount of the biomarker molecules present in said patient and therefore permits cancer diagnosis.

**14.** *In vitro* use of at least one probe as defined in any of the claims 1-6 or of the kit as defined in claim 7, for identifying new antibodies or new biological markers that can serve in immunotherapeutic treatments.

**15.** An *in vitro* method to screen and identify antibodies that can be efficient for immunotherapy or as chemotherapy drug carriers, said method comprising the following steps:

a) Contacting a sample comprising tumor cells with an effective amount of candidate antibodies that have been linked to a MS-tag by a linker as defined in claim 1, in appropriate conditions so that the candidate antibodies can bind efficiently to the target biomarker proteins, if present in the biological sample,
b) Contacting a sample comprising healthy cells with the same effective amount of the same candidate antibodies, in the same appropriate conditions as in step a),
c) Optionally, washing the samples in order to remove the unbound antibodies,
d) After step c), contacting the samples with an alkynyl cleaving compound as defined in claim 1 or 5, so as to release the MS-tags from the bound antibodies, if any,
e) Detecting the nature and optionally the amount of the cleaved MS-tags released in step d) by mass spectrometry, in the two samples,

wherein the nature and/or the amount of the cleaved MS-tags detected in step e) reveals the presence and / or the amount of the biomarker proteins specifically in the sample containing tumor cells and therefore the antibodies that detect cancer cells specifically.

Figure 1

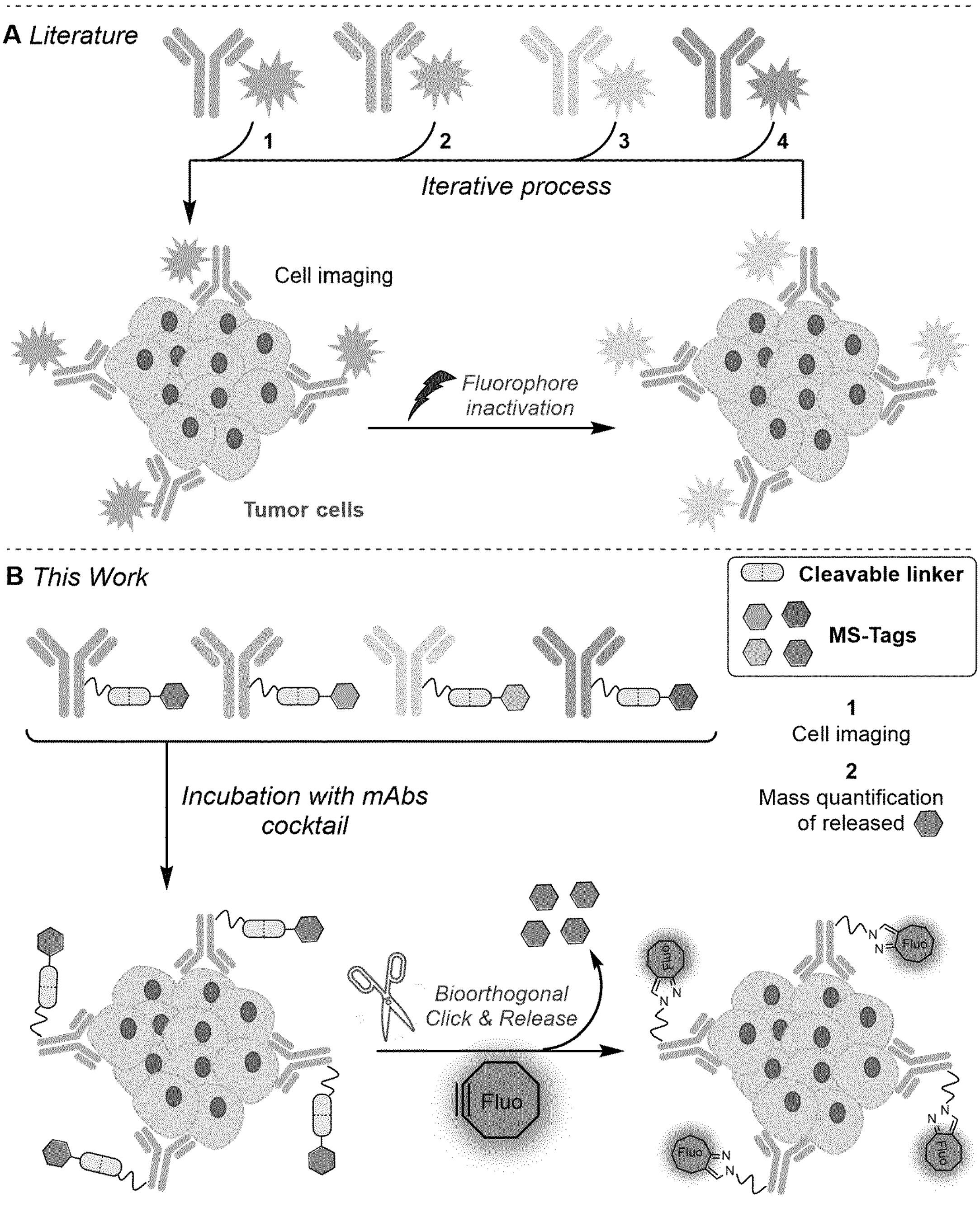
A Literature
1
2
3
4
Iterative process
Cell imaging
Fluorophore inactivation
Tumor cells
B This Work
Cleavable linker
MS-Tags
1
Cell imaging
2
Mass quantification of released
Incubation with mAbs cocktail
Bioorthogonal Click & Release
Fluo

## Figure 2

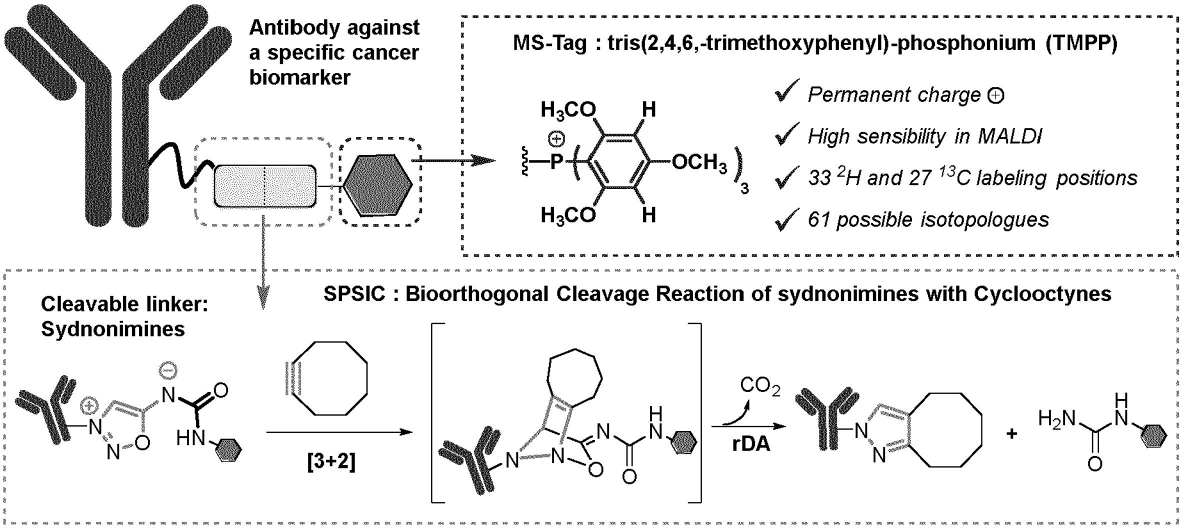


## Figure 3

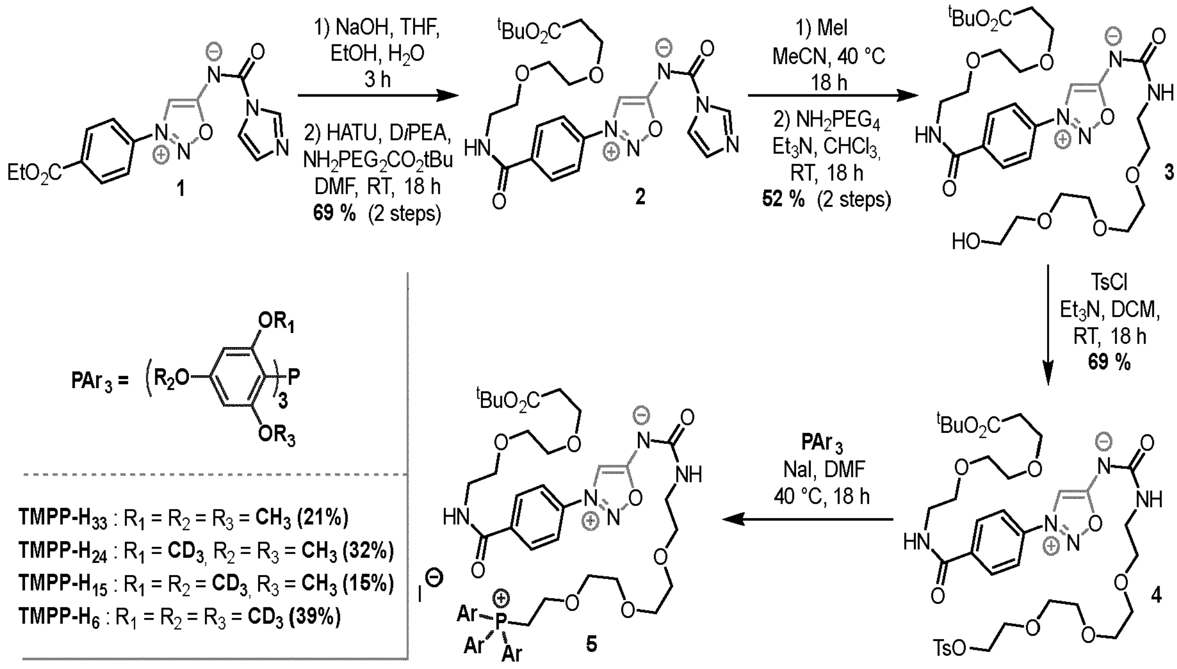

**Figure 4**

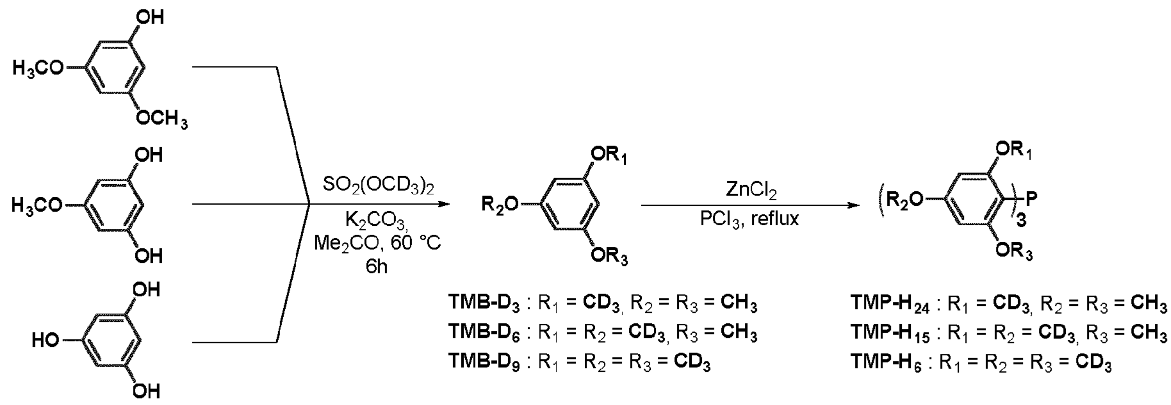


**TMB-D$_3$** : $R_1$ = **$CD_3$**, $R_2$ = $R_3$ = **$CH_3$**
**TMB-D$_6$** : $R_1$ = $R_2$ = **$CD_3$**, $R_3$ = **$CH_3$**
**TMB-D$_9$** : $R_1$ = $R_2$ = $R_3$ = **$CD_3$**

**TMP-H$_{24}$** : $R_1$ = **$CD_3$**, $R_2$ = $R_3$ = **$CH_3$**
**TMP-H$_{15}$** : $R_1$ = $R_2$ = **$CD_3$**, $R_3$ = **$CH_3$**
**TMP-H$_6$** : $R_1$ = $R_2$ = $R_3$ = **$CD_3$**

**Figure 5**

**A**

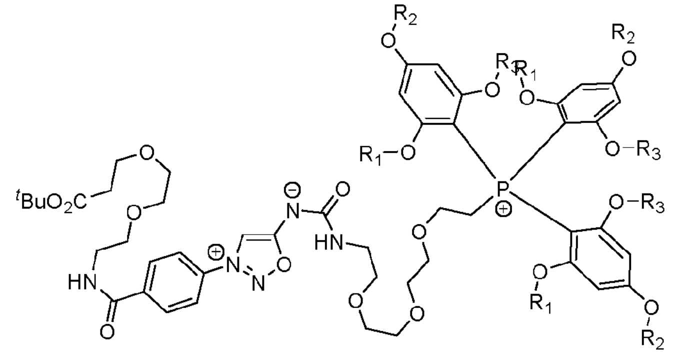

**5-H$_{33}$** : $R_1$ = $R_2$ = $R_3$ = **$CH_3$**
**5-H$_{24}$** : $R_1$ = **$CD_3$**, $R_2$ = $R_3$ = **$CH_3$**
**5-H$_{15}$** : $R_1$ = $R_2$ = **$CD_3$**, $R_3$ = **$CH_3$**
**5-H$_6$** : $R_1$ = $R_2$ = $R_3$ = **$CD_3$**

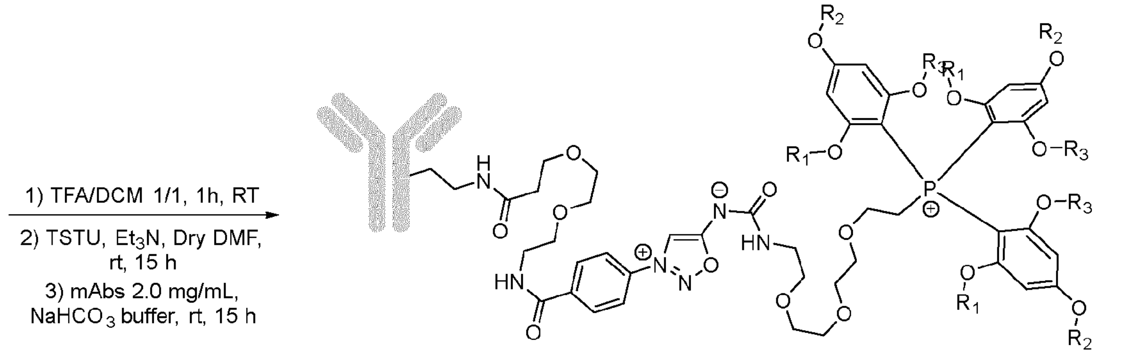


**TRZ-H$_{33}$** : $R_1$ = $R_2$ = $R_3$ = **$CH_3$**
**CTX-H$_{24}$** : $R_1$ = **$CD_3$**, $R_2$ = $R_3$ = **$CH_3$**
**DUR-H$_{15}$** : $R_1$ = $R_2$ = **$CD_3$**, $R_3$ = **$CH_3$**
**BVZ-H$_6$** : $R_1$ = $R_2$ = $R_3$ = **$CD_3$**

**Figure 5 (continuation)**

**B**

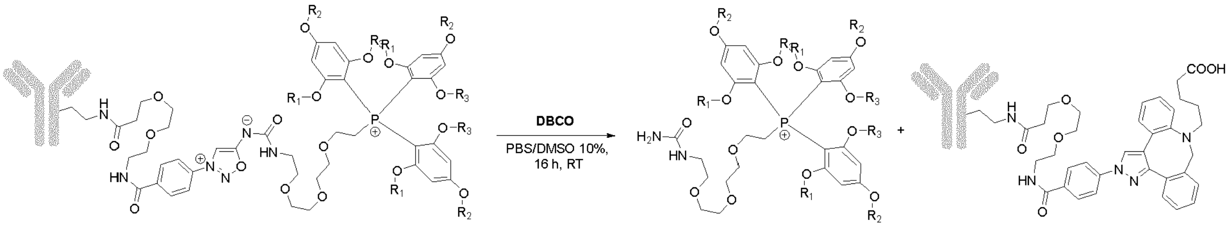


**TRZ-$H_{33}$** : $R_1 = R_2 = R_3 = CH_3$
**CTX-$H_{24}$** : $R_1 = CD_3$, $R_2 = R_3 = CH_3$
**DUR-$H_{15}$** : $R_1 = R_2 = CD_3$, $R_3 = CH_3$
**BVZ-$H_6$** : $R_1 = R_2 = R_3 = CD_3$

**TMPP-$H_{33}$** : $R_1 = R_2 = R_3 = CH_3$
**TMPP-$H_{24}$** : $R_1 = CD_3$, $R_2 = R_3 = CH_3$
**TMPP-$H_{15}$** : $R_1 = R_2 = CD_3$, $R_3 = CH_3$
**TMPP-$H_6$** : $R_1 = R_2 = R_3 = CD_3$

**Figure 6**

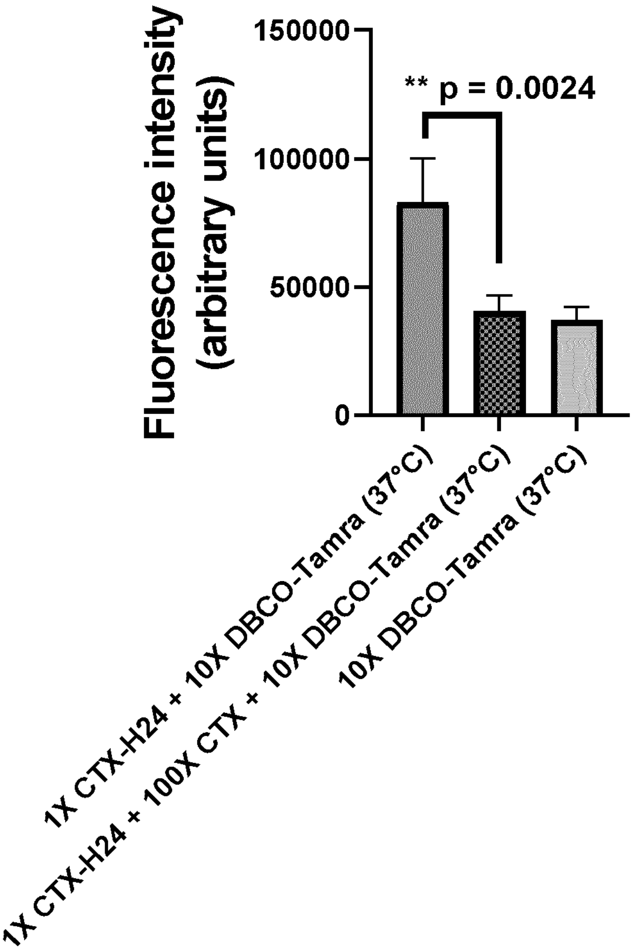

Figure 7

A

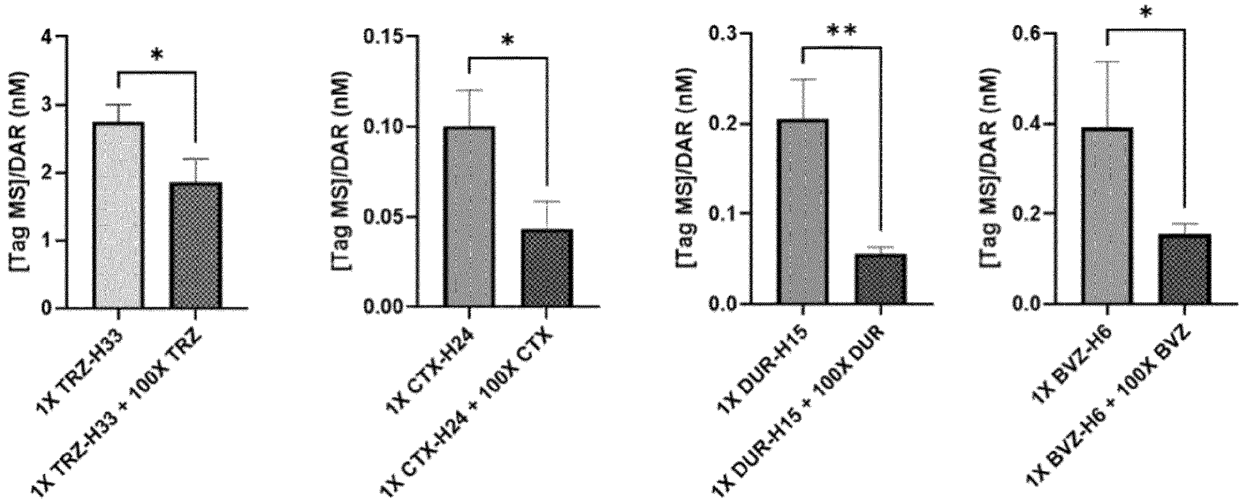
[Tag MS]/DAR (nM)
*
1X TRZ-H33
1X TRZ-H33 + 100X TRZ
*
1X CTX-H24
1X CTX-H24 + 100X CTX
**
1X DUR-H15
1X DUR-H15 + 100X DUR
*
1X BVZ-H6
1X BVZ-H6 + 100X BVZ

B

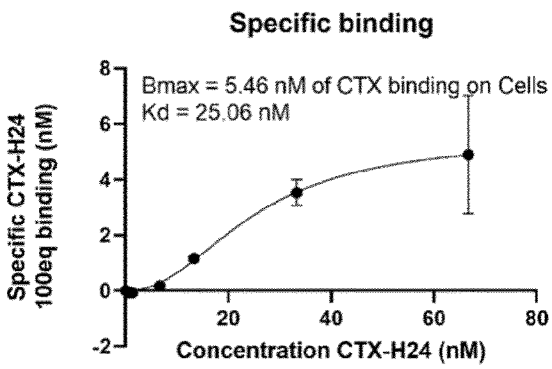
Specific binding
Bmax = 5.46 nM of CTX binding on Cells
Kd = 25.06 nM
Specific CTX-H24 100eq binding (nM)
Concentration CTX-H24 (nM)

C

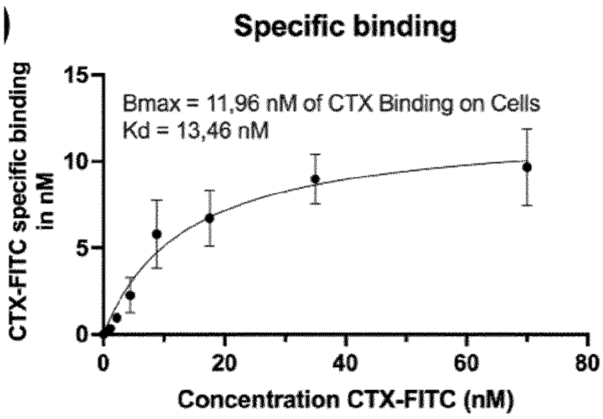
Specific binding
Bmax = 11,96 nM of CTX Binding on Cells
Kd = 13,46 nM
CTX-FITC specific binding in nM
Concentration CTX-FITC (nM)

**Figure 8**

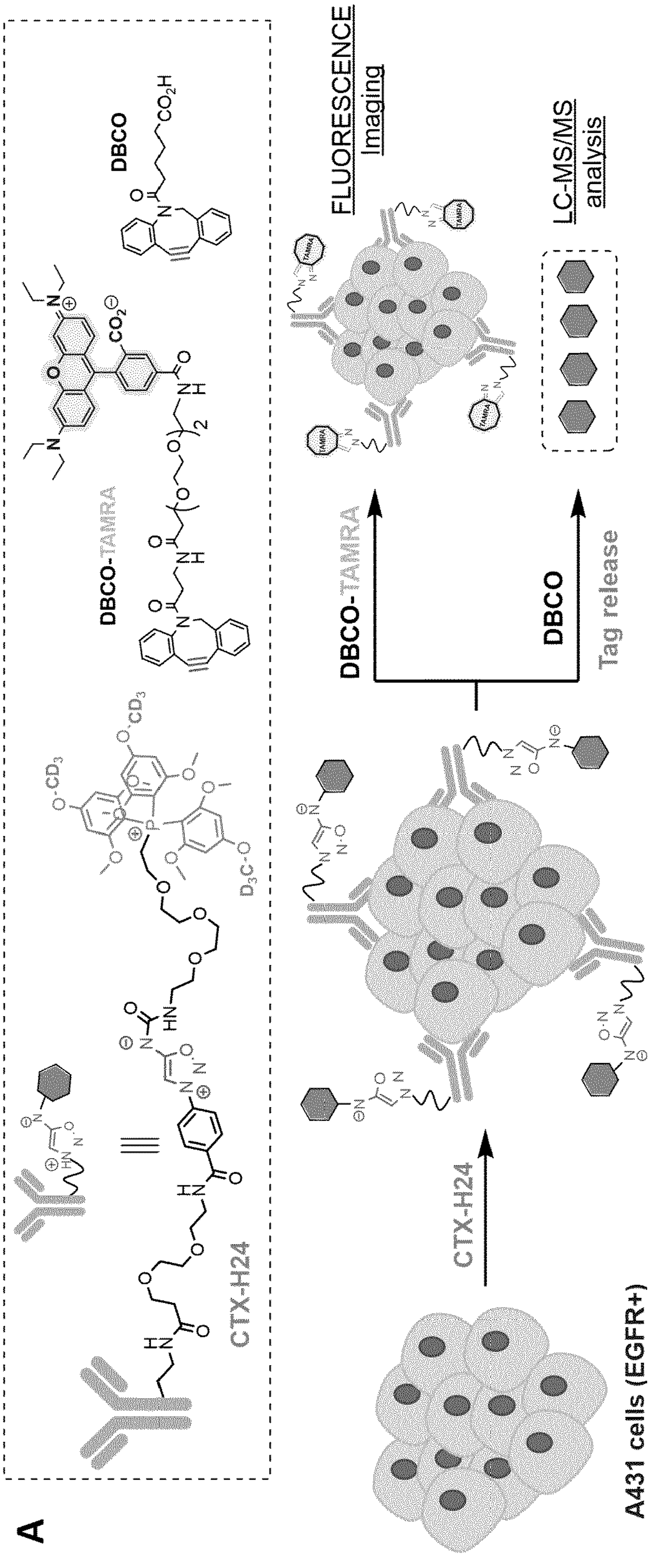
A
CTX-H24
DBCO-TAMRA
DBCO
FLUORESCENCE
Imaging
LC-MS/MS
analysis
Tag release
A431 cells (EGFR+)

Figure 8 (continuation)

B

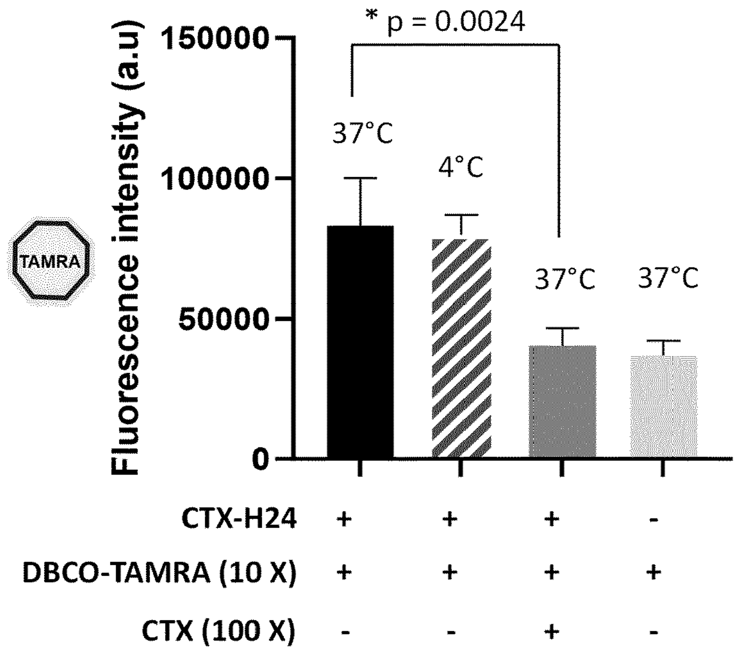


C

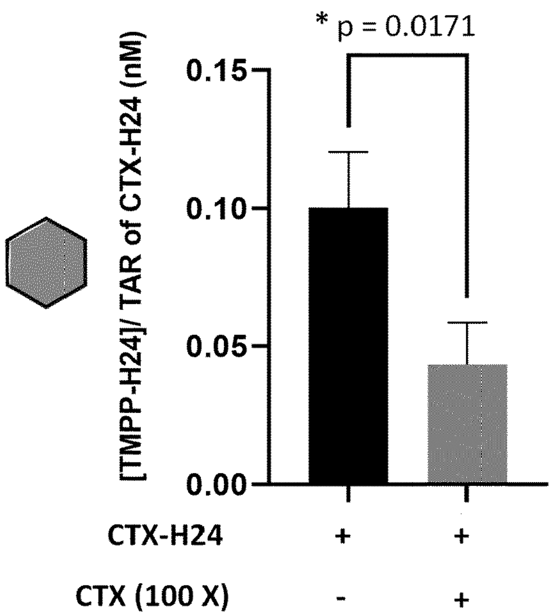

## Figure 9

A

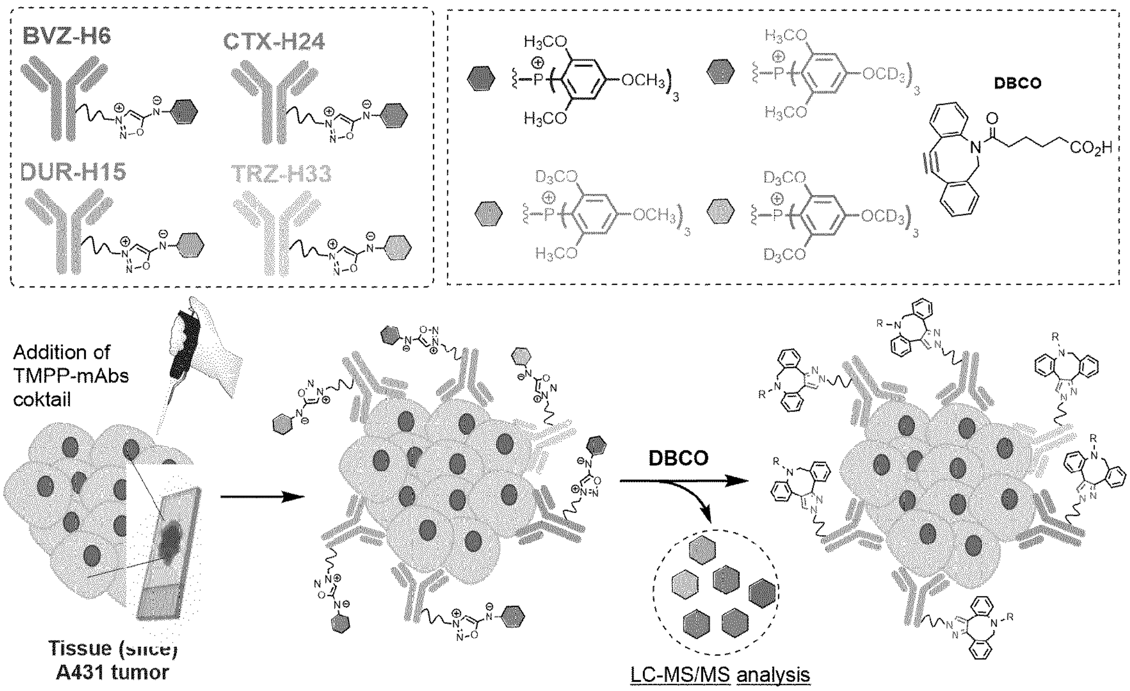


B

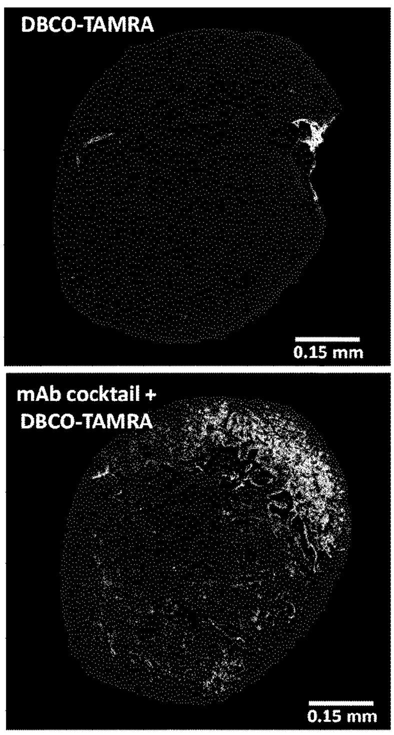


C

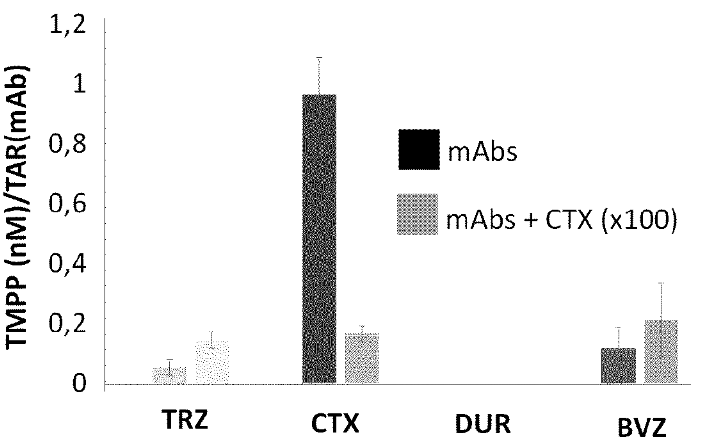


| mAb | Tag | Biomarker | Concentration | Abondance |
|---|---|---|---|---|
| TRZ | H33 | HER2 | 71,0 pM | 4,8 % |
| **CTX** | H24 | **EGFR** | **1265,9 pM** | **85,2 %** |
| DUR | H15 | PD-L1 | n.d. | n.d |
| BVZ | H6 | VEGF | 148,7 pM | 10,0 % |

Figure 10

A

*1) i.v. injection of the cocktail*

*2) intratumoral injection 3 days later*

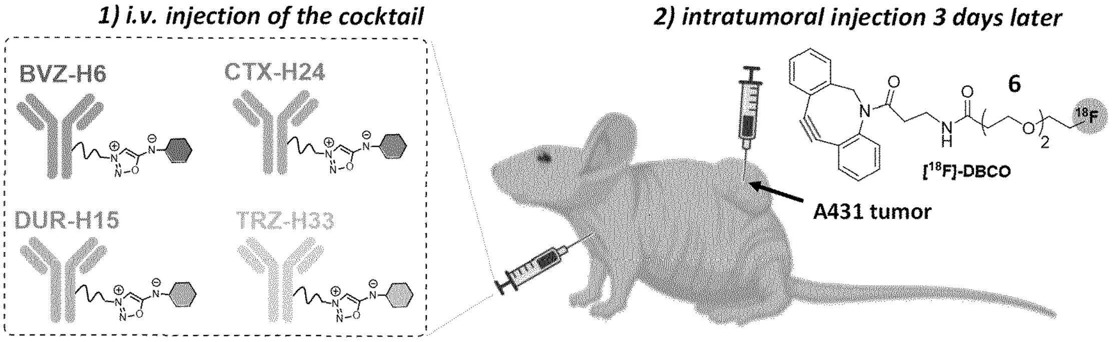


*3) LC-MS/MS of released tags in urines*

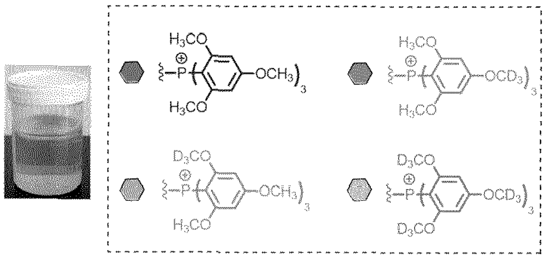

B)

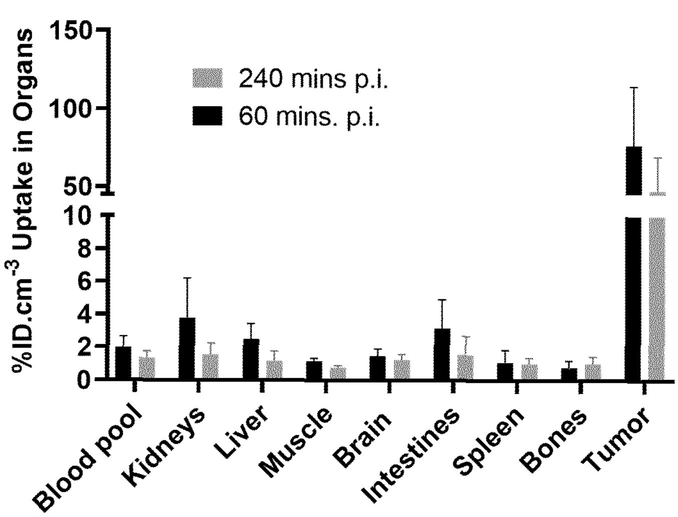


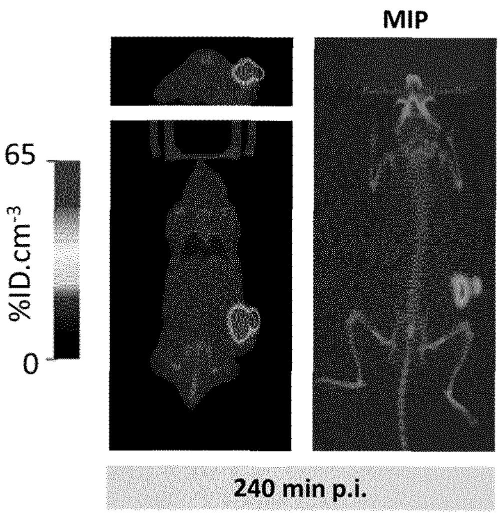

Figure 10 (continuation)

C

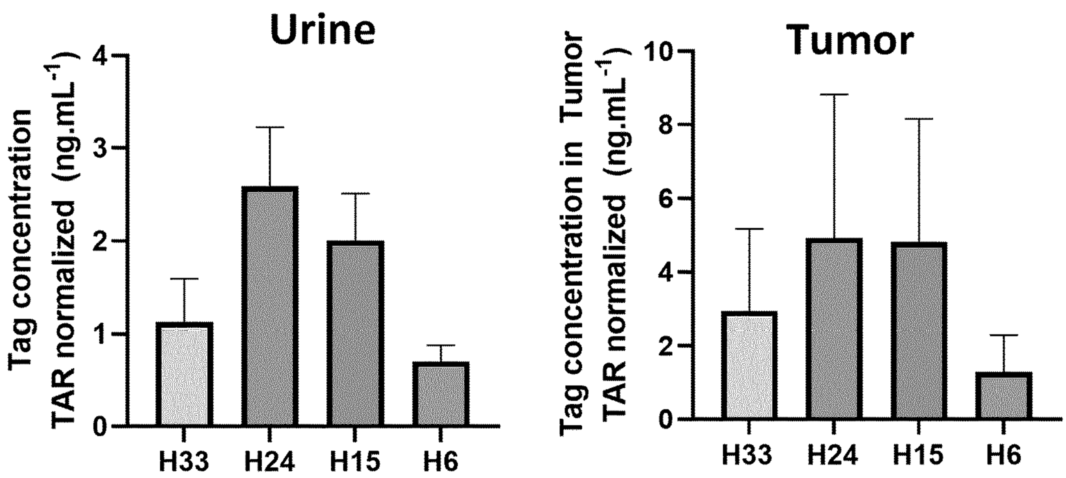

**Figure 11**

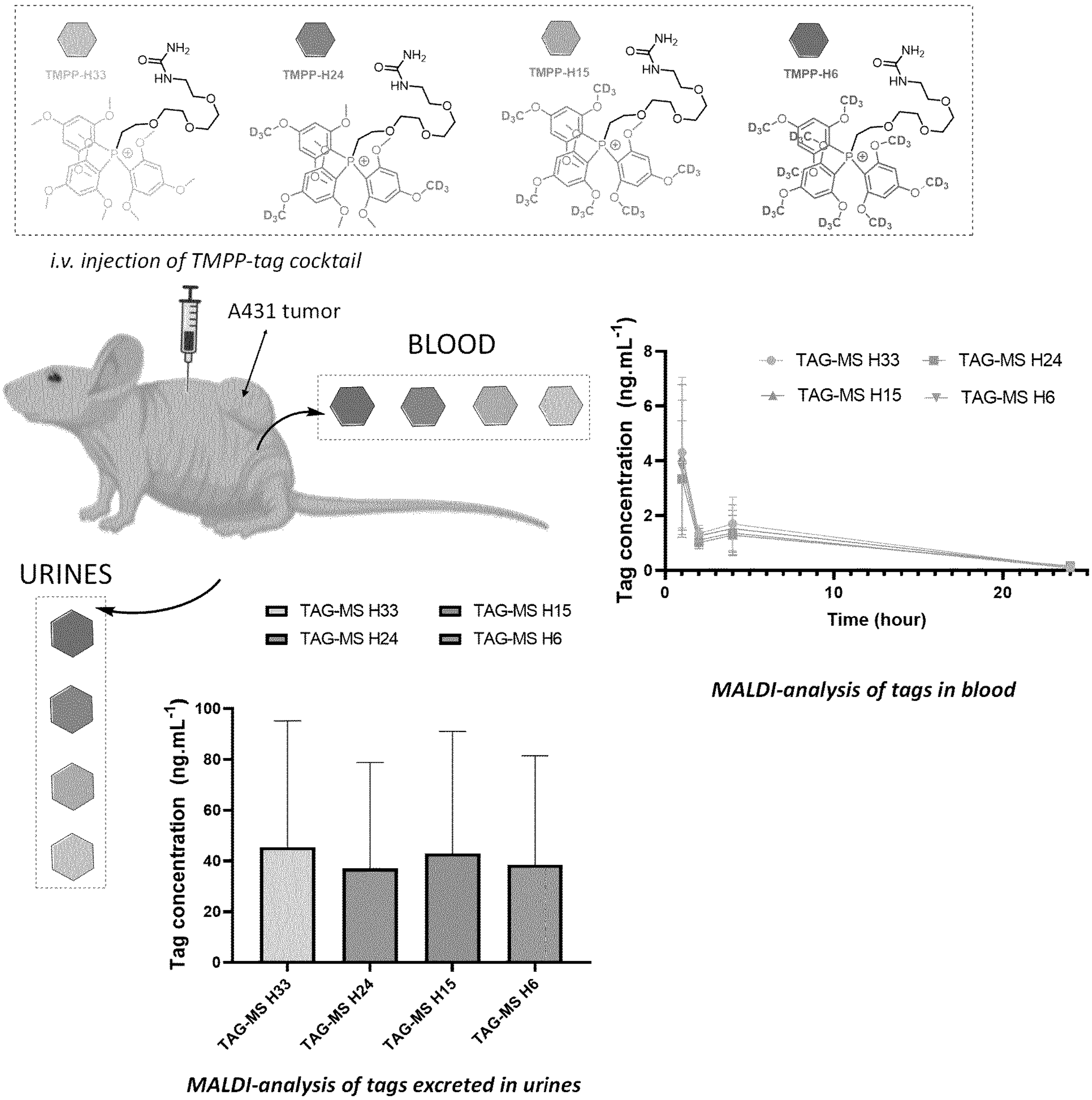
TMPP-H33
TMPP-H24
TMPP-H15
TMPP-H6
i.v. injection of TMPP-tag cocktail
A431 tumor
BLOOD
URINES
TAG-MS H33
TAG-MS H24
TAG-MS H15
TAG-MS H6
Tag concentration (ng.mL-1)
Time (hour)
MALDI-analysis of tags in blood
MALDI-analysis of tags excreted in urines

Figure 12

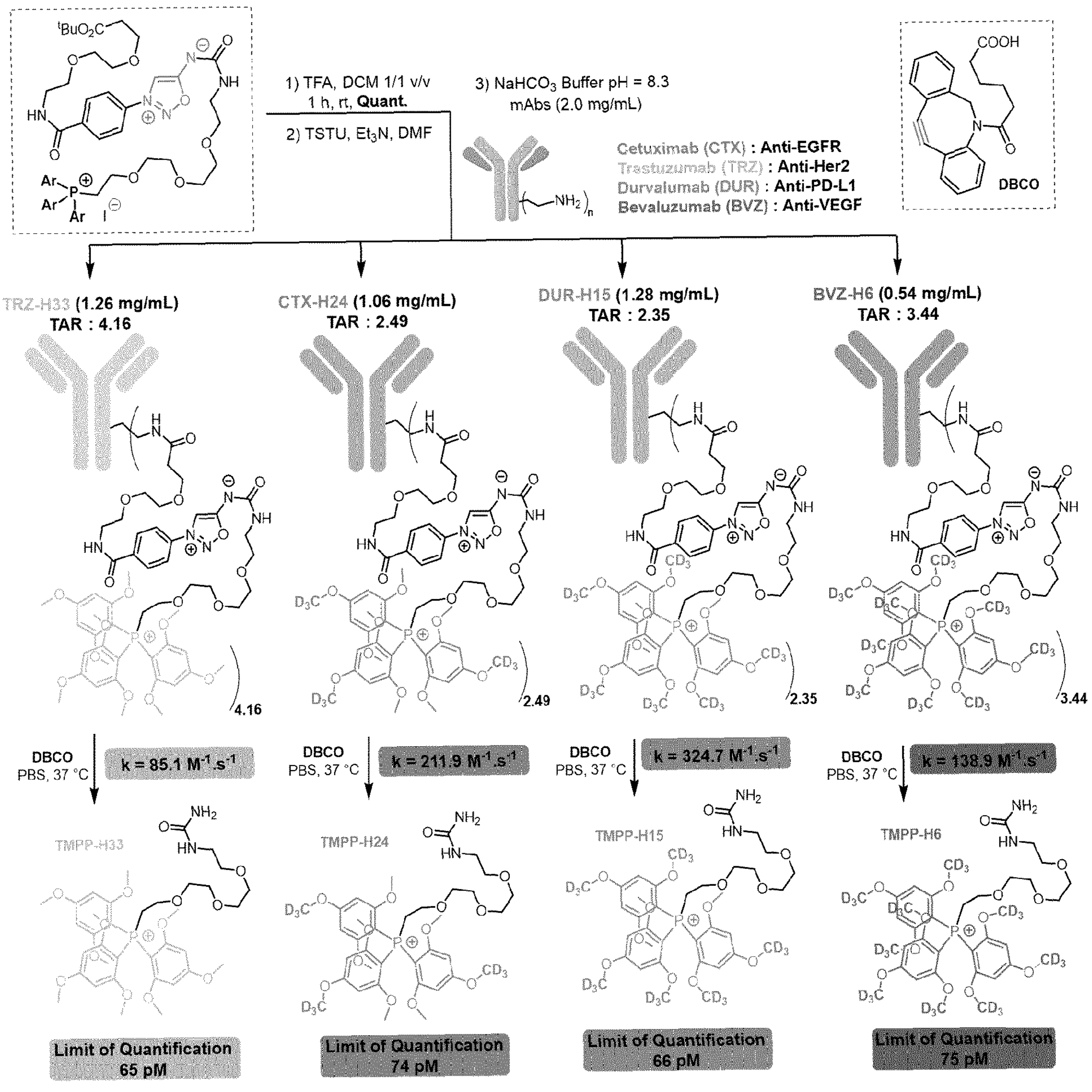
1) TFA, DCM 1/1 v/v
1 h, rt, Quant.
2) TSTU, $Et_3N$, DMF
3) $NaHCO_3$ Buffer pH = 8.3
mAbs (2.0 mg/mL)
Cetuximab (CTX) : Anti-EGFR
Trastuzumab (TRZ) : Anti-Her2
Durvalumab (DUR) : Anti-PD-L1
Bevaluzumab (BVZ) : Anti-VEGF
DBCO
TRZ-H33 (1.26 mg/mL)
TAR : 4.16
CTX-H24 (1.06 mg/mL)
TAR : 2.49
DUR-H15 (1.28 mg/mL)
TAR : 2.35
BVZ-H6 (0.54 mg/mL)
TAR : 3.44
DBCO
PBS, 37 °C
k = 85.1 $M^{-1}.s^{-1}$
k = 211.9 $M^{-1}.s^{-1}$
k = 324.7 $M^{-1}.s^{-1}$
k = 138.9 $M^{-1}.s^{-1}$
TMPP-H33
TMPP-H24
TMPP-H15
TMPP-H6
Limit of Quantification
65 pM
Limit of Quantification
74 pM
Limit of Quantification
66 pM
Limit of Quantification
75 pM

European Patent Office

# EUROPEAN SEARCH REPORT

Application Number
EP 24 30 5986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RIBÉRAUD MAXIME ET AL: "Fast and Bioorthogonal Release of Isocyanates in Living Cells from Iminosydnones and Cycloalkynes", JOURNAL OF THE AMERICAN CHEMICAL SOCIETY, vol. 145, no. 4, 19 January 2023 (2023-01-19), pages 2219-2229, XP093230714, ISSN: 0002-7863, DOI: 10.1021/jacs.2c09865 Retrieved from the Internet: URL:https://pubs.acs.org/doi/pdf/10.1021/jacs.2c09865> | 1,2,5,7,8 | INV. G01N33/68 C07D231/54 C07D271/04 C07D413/12 C07D495/04 C12Q1/6886 |
| Y | * page 2220, figure 1; page 2227, figure 5 * | 4,13-15 | |
| X | EP 2 957 559 A1 (UNIV STRASBOURG L [FR]; CENTRE NAT RECH SCIENT [FR] ET AL.) 23 December 2015 (2015-12-23) | 1-3,5-9 | |
| Y | * claims; paragraph [0039]; [0057]-[0064] * | 4,10-15 | |
| X | DU SHAOQING ET AL: "Discovery of novel iminosydnone compounds with insecticidal activities based on the binding mode of triflumezopyrim", BIOORGANIC & MEDICINAL CHEMISTRY LETTERS, ELSEVIER, AMSTERDAM NL, vol. 46, 17 May 2021 (2021-05-17), XP086646217, ISSN: 0960-894X, DOI: 10.1016/J.BMCL.2021.128120 [retrieved on 2021-05-17] | 1,2,8 | TECHNICAL FIELDS SEARCHED (IPC): G01N C07D C12Q |
| Y | * abstract; page 3, figure 4; page 4, table 2 * | 10-15 | |

-/--

The present search report has been drawn up for all claims

1

| Place of search | Date of completion of the search | Examiner |
|---|---|---|
| Munich | 4 December 2024 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 24 30 5986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAO YAN ET AL: "Bioorthogonal "Click and Release" Reaction-Triggered Aggregation of Gold Nanoparticles Combined with Released Lonidamine for Enhanced Cancer Photothermal Therapy", ANGEWANDTE CHEMIE INTERNATIONAL EDITION, VERLAG CHEMIE, HOBOKEN, USA, vol. 63, no. 13, 20 February 2024 (2024-02-20), page n/a, XP072604432, ISSN: 1433-7851, DOI: 10.1002/ANIE.202318539 | 1,2,5,8, 9 | |
| Y | * scheme 1; "Supporting information" * | 10-15 | |
| | -& Yan ET AL: "Bioorthogonal "Click and Release" Reaction-Triggered Aggregation of Gold Nanoparticles Combined with Released Lonidamine for Enhanced Cancer Photothermal Therapy", , 2 February 2024 (2024-02-02), pages 1-33, XP093230723, Retrieved from the Internet: URL:https://onlinelibrary.wiley.com/doi/10.1002/anie.202318539 | | |
| | ----- | | |
| X | SABRINA BERNARD ET AL: "Bioorthogonal Click and Release Reaction of Iminosydnones with Cycloalkynes", ANGEWANDTE CHEMIE INTERNATIONAL EDITION, VERLAG CHEMIE, HOBOKEN, USA, vol. 56, no. 49, 10 November 2017 (2017-11-10), pages 15612-15616, XP072092390, ISSN: 1433-7851, DOI: 10.1002/ANIE.201708790 | 1,2,5-8 | |
| Y | * page 15615, figure 2 * | 10-15 | |
| | ----- | | |
| | -/-- | | |

TECHNICAL FIELDS SEARCHED (IPC)

The present search report has been drawn up for all claims

1

| Place of search | Date of completion of the search | Examiner |
|---|---|---|
| Munich | 4 December 2024 | Hennard, Christophe |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 24 30 5986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SANTOS H M ET AL: "Probe-based chemical modulations of tissues for IMS", JOURNAL OF PROTEOMICS, vol. 75, no. 16, 25 May 2012 (2012-05-25), pages 4921-4930, XP028934853, ISSN: 1874-3919, DOI: 10.1016/J.JPROT.2012.05.025 * page 4924, figure 2; page 4922, item 2.1; page 4923, item 2.2 right-hand column * ----- | 4,10-15 | |

TECHNICAL FIELDS SEARCHED (IPC)

The present search report has been drawn up for all claims

1

| Place of search | Date of completion of the search | Examiner |
|---|---|---|
| Munich | 4 December 2024 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 24 30 5986

This annex lists the patent family members relating to the patent documents cited in the above-mentioned European search report. The members are as contained in the European Patent Office EDP file on
The European Patent Office is in no way liable for these particulars which are merely given for the purpose of information.

04-12-2024

| Patent document cited in search report | Publication date | Patent family member(s) | Publication date |
|---|---|---|---|
| EP 2957559 A1 | 23-12-2015 | EP 2957559 A1 | 23-12-2015 |
| | | EP 3157906 A1 | 26-04-2017 |
| | | US 2017129863 A1 | 11-05-2017 |
| | | US 2019144401 A1 | 16-05-2019 |
| | | WO 2015193455 A1 | 23-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015193455 A
- WO 9504160 A
- WO 9826095 A
- WO 9727327 A
- WO 9727325 A
- WO 9727331 A
- WO 0168664 A
- WO 03025576 A


### Non-patent literature cited in the description


- *J. Pharm. Sci.*, 1977, vol. 66 (2)
- **D. ZAHAVI** ; **L. WEINER**. *Antibodies*, 2020, vol. 9, 34
- **A. BECK** ; **L. GOETSCH** ; **C. DUMONTET** ; **N. CORVAÏA**. *Nature Reviews Drug Discovery*, 2017, vol. 16, 315
- **Y. IMAI** ; **C. K. LEUNG** ; **H. G. FRIESEN** ; **R. P. SHIU**. *Cancer Res.*, 1982, vol. 42, 4394
- **R. WEISSLEDER** ; **M. C. SCHWAIGER** ; **S. S. GAMBHIR** ; **H. HRICAK**. *Sci. Transl. Med*, 2016, vol. 8 (16), 355
- **S. C. BENDALL** ; **E. F. SIMONDS** ; **P. QIU** ; **EL-AD D** ; **AMIR, P. O.** ; **KRUTZIK, R.** ; **FINCK, R.** ; **V. BRUGGNER** ; **R. MELAMED** ; **A. TREJO**. *Science*, 2011, vol. 332, 687
- **W. SCHUBERT** ; **B. BONNEKOH** ; **A. J. POMMER** ; **L. PHILIPSEN** ; **R. BÇCKELMANN** ; **Y. MALYKH** ; **H. GOLLNICK** ; **M. FRIEDENBERGER** ; **M. BODE** ; **A. W. M. DRESS**. *Nat. Biotechnol.*, 2006, vol. 24, 1270
- **M. J. GERDES** ; **C. J. SEVINSKY** ; **A. SOOD** ; **S. ADAK** ; **M. O. BELLO** ; **A. BORDWELL** ; **A. CAN** ; **A. CORWIN** ; **S. DINN** ; **R. J. FILKINS**. *Proc. Natl. Acad. Sci. USA*, 2013, vol. 110, 11982
- **J. R. LIN** ; **M. FALLAHI-SICHANI** ; **P. K. SORGER**. *Nat. Commun.*, 2015, vol. 6, 8390
- **R. M. SCHWELLER** ; **J. ZIMAK** ; **D. Y. DUOSE** ; **A. A. QUTUB** ; **W. N. HITTELMAN** ; **M. R. DIEHL**. *Angew. Chem. Int. Ed.*, 2012, vol. 51, 9292
- **M. MONDAL** ; **R. LIAO** ; **L. XIAO** ; **T. ENO** ; **J. GUO**. *Angew. Chem. Int. Ed.*, 2017, vol. 56, 2636
- **J. KO** ; **J. OH** ; **M. S. AHMED** ; **J. C. T. CARLSON** ; **R. WEISSLEDER**. *Angew. Chem. Int. Ed.*, 2020, vol. 59, 6839
- **M. RIBÉRAUD** ; **E. PORRET A.** ; **PRUVOST, F. THEODORO** ; **A. L. NGUYEN** ; **S. SPECKLIN** ; **D. KERESELIDZE** ; **C. DENIS** ; **B. JEGO** ; **P. BARBE** ; **M. KECK**. *J. Am. Chem. Soc.*, 2023, vol. 145 (4), 2219-2229
- **TANG KC** ; **RAJ M.** *Angew. Chem. Int. Ed.*, 2021, vol. 60, 1797-1805
- **PARAMELLE D. et al.** *Angew. Chem. Int. Ed.*, 2010, vol. 49, 8240-8243
- **LI S. et al.** *Anal. Chem.*, 2019, vol. 91, 1701-1705
- **STEVENS KG** ; **PUKALA T.L**. *Trends in Analytical Chemistry*, 2020, vol. 132, 116064
- **GOYDEL RS** ; **RADER C.** *Oncogene*, May 2021, vol. 40 (21), 3655-3664
- **PASSARO A. et al.** *Cell*, vol. 187, 2024
- **RICHARD, M.** ; **TRUILLET, C.** ; **TRAN, V. L** ; **LIU, H.** ; **PORTE, K.** ; **AUDISIO, D.** ; **ROCHE, M** ; **JEGO, B** ; **CHOLET, S** ; **FENAILLE, F.** *Chem. Commun.*, 2019, vol. 55, 10400-10403
- **M. RIOMET** ; **K. PORTE** ; **L. MADEGARD** ; **P. THUÉRY** ; **D. AUDISIO** ; **F. TARAN**. *Org. Lett.*, 2020, vol. 22 (6), 2403
- **M. RIOMET** ; **E. DECUYPERE** ; **K. PORTE** ; **S. BERNARD** ; **L. PLOUGASTEL** ; **S. KOLODYCH** ; **D. AUDISIO** ; **F. TARAN**. *Chem. Eur. J.*, 2018, vol. 34, 8535
- **S. BERNARD** ; **D. AUDISIO** ; **M. RIOMET** ; **S. BREGANT** ; **A. SALLUSTRAU** ; **L. PLOUGASTEL** ; **E. DECUYPERE** ; **S. GABILLET** ; **R. A. KUMAR** ; **J. ELYIAN**. *Angew. Chem. Int. Ed.*, 2017, vol. 56, 15612
- **W. B. NAGENGAST** ; **M. N. LUB-DE HOOGE** ; **S. F. OOSTING** ; **W. F. A. DEN DUNNEN** ; **F-J. WARNDERS** ; **A. H. BROUWERS** ; **J. R. DE JONG** ; **P. M. PRICE** ; **H. HOLLEMA** ; **G. A. P. HOSPERS**. *Cancer Research*, 2011, vol. 71, 143
- **D. PATEL** ; **A. LAHIJI** ; **S. PATEL** ; **M. FRANKLIN** ; **X. JIMENEZ** ; **D. J. HICKLIN** ; **X. KANG**. *Anticancer Res.*, 2007, vol. 27 (5A), 3355
- **PROTOPOPOV**. *Obshch. Khim.*, 1963, vol. 33, 3050-3052